# EUROPÄISCHE PATENTSCHRIFT

(11) **EP 0 733 642 B1**
(45) Veröffentlichungstag und Bekanntmachung des Hinweises auf die Patenterteilung: **29.11.2000**
(21) Anmeldenummer: 96104265.2
(22) Anmeldetag: 18.03.1996
(51) Int. Cl.: C07K 5/078, A61K 38/05

(54) **Benzazepin-, Benzoxazepin- und Benzothiazepin-N-essigsäure-derivate sowie Verfahren zu ihrer Herstellung und diese Verbindungen enthaltende Arzneimittel**
Benzazepin-, benzoxazepin- and benzothiazepin-N-acetic acid-derivatives, their preparation and their pharmaceutical compositions
Dérivés d'acide benzazepine-, benzoxazepine- et benzothiazepine-N-acétique, leur préparation et des composés les contenant

(30) Priorität: 23.03.1995 DE 19510566
(43) Veröffentlichungstag der Anmeldung: 25.09.1996
(73) Patentinhaber: Solvay Pharmaceuticals GmbH, 30173 Hannover (DE)
(72) Erfinder: Waldeck, Harald, 30916 Isernhagen HB (DE); Höltje, Dagmar, 30989 Gehrden (DE); Messinger, Josef, 31319 Sehnde (DE); Antel, Jochen, 31848 Bad Münder (DE); Wurl, Michael, 30826 Garbsen (DE); Thormählen, Dirk, 31039 Rheden (DE)
(74) Vertreter: Lauer, Dieter, Dr.

(56) Entgegenhaltungen:
- EP-A- 0 534 396
- EP-A- 0 595 610
- EP-A- 0 599 444
- EP-A- 0 636 621
- EP-A- 0 636 630
- BIOCHEMICAL AND BIOPHYSICAL RESEARCH COMMUNICATIONS, Bd. 204, Nr. 1, 14.Oktober 1994, ORLANDO, FL US, Seiten 407-412, XP002008404 S.DE LOMBAERT E.A.: "Pharmacological profile of a non-peptide dual inhibitor of NEP and ECE"

## Beschreibung

Die vorliegende Erfindung betrifft neue Benzazepin-, Benzoxazepin- und Benzothiazepin-N-essigsäurederivate, welche in α-Stellung zu dem Stickstoffatom eine Oxogruppe enthalten und in 3-Stellung durch einen 1-(Carboxyalkyl)-cyclopentylcarbonyl-amino-Rest substituiert sind, und deren Salze und biolabile Ester sowie diese Verbindungen enthaltende pharmazeutische Zubereitungen und Verfahren zur Herstellung dieser Verbindungen.

Aus den europäischen Patentanmeldungen EP-A 0 636 621 und EP-A 0 636 630 sowie aus der Veröffentlichung von Lombaert et al. in der Zeitschrift Biochem. Biophys. Res. Comm., Bd. 204, Nr. 1, 1994, Seiten 407-412, sind bereits Verbindungen bekannt, welche eine inhibitorische Wirkung auf die neutrale Endopeptidase (= NEP) und das Endothelin convertierende Enzym (= ECE) besitzen. Die in den vorgenannten Veröffentlichungen enthaltenen Verbindungen unterscheiden sich von den Verbindungen der vorliegenden Erfindung jedoch durch wesentliche strukturelle Merkmale.

Weiterhin werden in den europäischen Patentanmeldungen EP-A 0 534 396, EP-A 0 595 610 und EP-A 0 599 444 Benzazepin-Derivate mit zu den Verbindungen der vorliegenden Erfindung jeweils ähnlichen Strukturen offenbart, welche inhibitorische Wirkungen auf das Angiotensin convertierende Enzym ( = ACE) und auf die NEP besitzen. Keiner der drei vorgenannten europäischen Patentanmeldungen war jedoch zu entnehmen, daß Benzazepin-Verbindungen der darin beschriebenen Art auch inhibitorische Wirkungen auf das ECE besitzen würden.

Der vorliegenden Erfindung liegt die Aufgabe zugrunde, neue Benzazepin-, Benzoxazepin- und Benzothiazepin-Verbindungen mit wertvollen pharmakologischen Eigenschaften zu entwickeln. Ferner liegt der Erfindung die Aufgabe zugrunde, neue zur Behandlung von Herzinsuffizienz einsetzbare pharmazeutische Wirkstoffe zu entwickeln.

Es wurde nun gefunden, daß die erfindungsgemäßen neuen in 3-Stellung einen gegebenenfalls veresterten 1-(Carboxyalkyl)-cyclopentylcarbonylamino-Rest tragenden Benzazepin-, Benzoxazepin- und Benzothiazepin-N-essigsäurederivate wertvolle herzwirksame pharmakologische Eigenschaften besitzen und eine ausgeprägte Hemmwirkung auf die neutrale Endopeptidase mit einem günstigen Wirkungsprofil aufweisen, aufgrund deren sie den bei Herzinsuffizienz auftretenden hohen kardialen Füllungsdruck reduzieren und so das Herz entlasten und eine Diurese-Verstärkung bewirken können.

Die Erfindung betrifft daher neue Verbindungen der allgemeinen Formel I
(siehe Formel I)
worin
- R¹: für eine C₁-C₄-Alkoxy-C₁-C₄-alkylgruppe, deren C₁-C₄-Alkoxyrest durch eine C₁-C₄-Alkoxygruppe substituiert ist, eine Phenyl-C₁-C₄-alkyl- oder Phenyloxy-C₁-C₄-alkylgruppe, welche gegebenenfalls im Phenylring durch C₁-C₄-Alkyl, C₁-C₄-Alkoxy oder Halogen substituiert sein kann, oder eine Naphthyl-C₁-C₄-alkylgruppe steht,
- A: für CH₂, O oder S steht,
- R²: Wasserstoff oder Halogen bedeutet,
- R³: Wasserstoff oder Halogen bedeutet,
- R⁴: Wasserstoff oder eine einen biolabilen Ester bildende Gruppe bedeutet und
- R⁵: Wasserstoff oder eine einen biolabilen Ester bildende Gruppe bedeutet
und physiologisch verträgliche Salze der Säuren der Formel I.

Sofern in den Verbindungen der Formel I die Substituenten niedere Alkyl- oder Alkoxygruppen bedeuten oder enthalten, können diese geradkettig oder verzweigt sein und, wenn nicht anders definiert, 1 bis 4, vorzugsweise 1 bis 2, Kohlenstoffatome enthalten und stellen bevorzugt Methyl oder Methoxy dar. Sofern die Substituenten Halogen bedeuten oder Halogensubstituenten enthalten, kommen insbesondere Fluor, Chlor oder Brom, vorzugsweise Fluor oder Chlor in Frage.

In den Verbindungen der Formel I kann A für eine Methylengruppe, Sauerstoff oder Schwefel stehen und stellt vorzugsweise Methylen dar.

Die Verbindungen der Formel I können in dem Phenylring die Substituenten R² und R³ tragen. Vorzugsweise stehen beide Substituenten R² und R³ oder zumindest jedoch einer dieser Substituenten für Wasserstoff.

R¹ stellt vorzugsweise einen einen aromatischen Ring enthaltenden Rest dar, beispielsweise einen gegebenenfalls substituierten Phenylniederalkyl- oder Phenyloxyniederalkylrest, in welchem die Niederalkylenkette 1 bis 4, vorzugsweise 1 bis 2 Kohlenstoffatome enthalten kann. Insbesondere stellt R¹ eine gegebenenfalls substituierte Phenethylgruppe dar, welche gegebenenfalls ein- oder mehrfach durch Halogen, niederes Alkoxy oder niederes Alkyl substituiert sein kann oder eine Naphthylethylgruppe. Sofern R¹ eine durch niederes Alkoxy substituierte Niederalkoxyniederalkylgruppe bedeutet, stellt diese vorzugsweise eine Niederalkoxymethylgruppe dar, worin der Niederalkoxyrest 1 bis 4, vorzugsweise 1 bis 2 Kohlenstoffatome enthält und durch niederes Alkoxy, insbesondere Methoxy substituiert ist.

Die Verbindungen der Formel I stellen gegebenenfalls veresterte Dicarbonsäurederivate dar. Je nach Applikationsform sind biolabile Monoester, insbesondere Verbindungen, worin R⁴ eine einen biolabilen Ester bildende Gruppe und R⁵ Wasserstoff bedeuten, oder Dicarbonsäuren bevorzugt, wobei letztere insbesondere für i.v.-Applikation geeignet sind.

Als biolabile Ester bildende Gruppen R⁴ und R⁵ eignen sich C₁₋₄ Alkylgruppen, gegebenenfalls im Phenylring durch C₁₋₄ Alkyl oder durch eine an zwei benachbarte Kohlenstoffatome gebundene C₃₋₄ Alkylenkette substituierte Phenyl- oder Phenyl C₁-₃ alkylgruppe, im Dioxolanring gegebenenfalls durch C₁-₄-Alkyl substituierte Dioxolanylmethylgruppen insbesondere (2,2-Dimethyl-1,3-dioxolan-4-yl)methyl, oder gegebenenfalls an der Oxymethylgruppe durch C₁₋₄-Alkyl substituierte C₂-C₆-Alkanoyloxymethylgruppen. Sofern die einen biolabilen Ester bildende Gruppe R⁴ oder R⁵ niederes Alkyl bedeutet, kann dieses eine bevorzugt unverzweigte Alkylgruppe mit 1 bis 4, vorzugsweise 2 Kohlenstoffatomen darstellen. Sofern die einen biolabilen Ester bildende Gruppe eine gegebenenfalls substituierte Phenylniederalkylgruppe darstellt, kann deren Alkylkette 1 bis 3, vorzugsweise 1, Kohlenstoffatome enthalten. Sofern der Phenylring durch eine niedere Alkylenkette substituiert ist, kann diese 3 bis 4, insbesondere 3 Kohlenstoffatome enthalten. Als phenylhaltige Substituenten R⁴ und/ oder R⁵ eignen sich insbesondere Phenyl, Benzyl oder Indanyl. Sofern R⁴ und/oder R⁵ eine gegebenenfalls substituierte Alkanoyloxymethylgruppe darstellen, kann deren Alkanoyloxygruppe 2 bis 6, vorzugsweise 3 bis 5, Kohlenstoffatome enthalten und ist vorzugsweise verzweigt und kann beispielsweise einen Pivaloyloxymethylrest (= tert.-Butylcarbonyloxymethylrest) darstellen.

Erfindungsgemäß werden die neuen Verbindungen der Formel I und deren Salze erhalten, indem man in an sich bekannter Weise
Säuren der allgemeinen Formel II
(siehe Formel II)
worin R¹ obige Bedeutung besitzt und R^{4a} eine Säureschutzgruppe bedeutet, oder deren reaktionsfähige Säurederivate mit Aminen der allgemeinen Formel III
(siehe Formel III)
worin R², R³ und A obige Bedeutung besitzen und R^{5a} eine Säureschutzgruppe bedeutet, zu Amiden der allgemeinen Formel IV
(siehe Formel IV)
worin R¹, R², R³, R^{4a}, R^{5a} und A obige Bedeutung besitzen, umsetzt und in den Verbindungen der Formel IV die Säureschutzgruppen R^{4a} und R^{5a}, sofern diese nicht eine gewünschte einen biolabilen Ester bildende Gruppe darstellen, gleichzeitig oder in beliebiger Reihenfolge nacheinander abspaltet und gewünschtenfalls jeweils die freiwerdende Säuregruppe mit einem Alkohol der allgemeinen Formel V oder einem entsprechenden reaktiven Derivat der allgemeinen Formel Va
(siehe Formeln V und Va)
worin R⁶ eine einen biolabilen Ester bildende Gruppe darstellt und X eine abspaltbare reaktive Gruppe bedeutet, verestert,
und gewünschtenfalls erhaltene Säuren der Formel I in ihre physiologisch verträglichen Salze überführt oder Salze der Säuren der Formel I in die freien Säuren überführt.

Als physiologisch verträgliche Salze von Dicarbonsäuren oder Monoestern der Formel I kommen deren Alkalimetall-, Erdalkalimetall- oder Ammoniumsalze in Frage, beispielsweise Natrium- oder Kalziumsalze oder Salze mit physiologisch verträglichen, pharmakologisch neutralen organischen Aminen wie beispielsweise Diethylamin oder tert.-Butylamin.

Die Verbindungen der Formel I enthalten zwei chirale Kohlenstoffatome, nämlich das die Amidseitenkette tragende Kohlenstoffatom in 3-Stellung des Ringgerüstes und das den Rest R¹ tragende Kohlenstoffatom der Amidseitenkette. Die Verbindungen können somit in mehreren optisch aktiven stereoisomeren Formen oder als Racemat vorliegen. Die vorliegende Erfindung umfaßt sowohl die racemischen Gemische wie auch die isomerenreinen Verbindungen der Formel I.

Die Umsetzung der Säuren der Formel II mit den Aminen der Formel III zu den Amiden der Formel IV kann nach an sich zur Bildung von Amidgruppierungen durch Aminoacylierung üblichen Methoden ausgeführt werden. Als Acylierungsmittel können die Säuren der Formel II oder deren reaktionsfähige Derivate eingesetzt werden. Als reaktionsfähige Derivate kommen insbesondere gemischte Säureanhydride und Säurehalogenide in Frage. So können beispielsweise Säurechloride oder Säurebromide der Säuren der Formel II oder gemischte Ester der Säuren der Formel II mit organischen Sulfonsäuren, beispielsweise Niederalkansulfonsäuren wie z. B. Methansulfonsäure oder aromatischen Sulfonsäuren wie z. B. Benzolsulfonsäure oder durch niederes Alkyl oder Halogen substituierten Benzolsulfonsäuren z. B. Toluolsulfonsäuren oder Brombenzolsulfonsäuren eingesetzt werden. Die Acylierung kann in einem unter den Reaktionsbedingungen inerten organischen Lösungsmittel, vorzugsweise bei Temperaturen zwischen -20 °C und Raumtemperatur erfolgen. Als Lösungsmittel eignen sich insbesondere halogenierte Kohlenwasserstoffe wie Dichlormethan oder aromatische Kohlenwasserstoffe wie Benzol oder Toluol oder cyclische Ether wie Tetrahydrofuran oder Dioxan oder Gemische dieser Lösungsmittel.

Die Acylierung kann zweckmäßig, insbesondere wenn als Acylierungsmittel ein gemischtes Anhydrid der Säuren der Formel II mit einer Sulfonsäure verwendet wird, in Gegenwart eines säurebindenden Reagenzes durchgeführt werden. Als säurebindende Mittel eignen sich in dem Reaktionsgemisch lösliche Basen, insbesondere organische Basen wie tert. Niederalkylamine und Pyridine wie z. B. Triethylamin, Tripropylamin, Pyridin, 4-Dimethylaminopyridin, 4-Diethylaminopyridin oder 4-Pyrrolidinopyridin. Im Überschuß eingesetzte organische Basen können gleichzeitig auch als Lösungsmittel dienen.

Vorteilhaft können gemischte Säureanhydride der Säuren der Formel II mit organischen Sulfonsäuren in situ durch Umsetzen der Säuren der Formel II mit einem Säurehalogenid, insbesondere dem Säurechlorid, der organischen Sulfonsäure erhalten werden und ohne Isolierung direkt weiter mit der Aminverbindung der Formel III umgesetzt werden.

Falls als Acylierungsmittel die Säuren der Formel II selbst eingesetzt werden, kann die Umsetzung der Aminoverbindungen der Formel III mit den Säuren der Formel II zweckmäßig auch in Gegenwart eines aus der Peptidchemie als zur Amidbildung geeignet bekannten Kopplungsreagenzes durchgeführt werden. Als Beispiel von Kopplungsreagenzien, welche die Amidbildung mit den freien Säuren dadurch fördern, daß sie mit der Säure in situ unter Bildung eines reaktionsfähigen Säurederivates reagieren, seien insbesondere genannt Alkylcarbodiimide, z. B. Cycloalkylcarbodiimide wie Dicyclohexylcarbodiimid oder 1-Ethyl-3-[3-(dimethylamino)-propyl]-carbodiimid, Carbonyldiimidazol und N-Niederalkyl-2-halogenpyridiniumsalze, insbesondere Halogenide oder Tosylate, vorzugsweise N-Methyl-2-chlorpyridinium-jodid (siehe z. B. Mukaijama in Angewandte Chemie 91, Seiten 789 - 812). Die Umsetzung in Gegenwart eines Kopplungsreagenzes kann zweckmäßig bei Temperaturen von -30 bis +50 °C unter Benutzung von Lösungsmitteln wie halogenierten Kohlenwasserstoffen und/oder aromatischen Lösungsmitteln, gegebenenfalls in Gegenwart eines säurebindenden Amins durchgeführt werden.

Aus den durch Umsetzung der Verbindungen der Formel II mit den Verbindungen der Formel III erhaltenen Verbindungen der Formel IV können die Schutzgruppen R^{4a} und R^{5a}, sofern sie keine in den Verbindungen der Formel I gewünschten, einen biolabilen Ester bildende Gruppen darstellen, auf an sich bekannte Weise abgespalten werden.

Als Schutzgruppen R^{4a} und R^{5a} können an sich zum Schutz von Säurefunktionen übliche Schutzgruppen gewählt werden, die anschließend nach an sich bekannten Methoden wieder abgespalten werden. Geeignete Säureschutzgruppen sind beispielsweise bekannt aus McOmie, "Protective Groups in Organic Chemistry", Plenum Press und Greene, "Protective Groups in Organic Synthesis" Wiley Intersience Publication.

Sofern Verbindungen der Formel I hergestellt werden sollen, in denen R⁴ und R⁵ identisch sind, werden zweckmäßigerweise identische Schutzgruppen R^{4a} und R^{5a} in den Ausgangsverbindungen II und III gewählt.

Sofern Verbindungen der Formel I hergestellt werden sollen, worin R⁴ und R⁵ unterschiedliche Bedeutung besitzen, werden zweckmäßigerweise in den Ausgangsverbindungen II und III unterschiedliche Schutzgruppen gewählt, welche bei unterschiedlichen Bedingungen auf an sich bekannte Weise selektiv wieder abgespalten werden können. Als Beispiele dreier unter unterschiedlichen Bedingungen abspaltbarer Schutzgruppen seien genannt:
1. Methyl- oder Ethylester, welche unter basischen Bedingungen leicht gespalten werden, jedoch gegen saure Bedingungen oder Hydrogenolyse wesentlich stabiler sind,
2. tert.-Butylester, welche leicht durch Säuren gespalten werden können, jedoch gegen basische Bedingungen oder Hydrogenolyse wesentlich stabiler sind und
3. Benzylester, welche leicht hydrogenolytisch oder auch unter basischen Bedingungen gespalten werden können, jedoch gegenüber sauren Bedingungen wesentlich stabiler sind.

Falls beispielsweise Dicarbonsäure-Verbindungen der Formel I hergestellt werden sollen, worin R⁴ und R⁵ beide Wasserstoff sind, werden als Schutzgruppen R^{4a} und R^{5a} bevorzugt sauer abspaltbare Schutzgruppen, beispielsweise die tert.-Butylgruppe, eingesetzt und die durch Umsetzung der Verbindungen der Formel II mit den Verbindungen der Formel III erhaltenen tert.-Butylester-Verbindungen der Formel IV anschließend durch Behandlung mit Säure gespalten. Die Spaltung kann z. B. durch Behandeln mit Trifluoressigsäure als solcher oder einer Trifluoressigsäurelösung in einem halogenierten Kohlenwasserstoff, beispielsweise Dichlormethan, oder durch Behandeln mit HCl-Gas in einem unter den Reaktionsbedingungen inerten organischen Lösungsmittel, beispielsweise Essigsäureethylester, erfolgen. Die Reaktion kann bei Temperaturen zwischen -25 °C und Raumtemperatur durchgeführt werden.

Falls beispielsweise Monocarbonsäure-Verbindungen der Formel I hergestellt werden sollen, worin R⁴ eine einen biolabilen Ester bildende Gruppe bedeutet und R⁵ Wasserstoff ist, können als Ausgangsverbindungen der Formel II Verbindungen eingesetzt werden, in denen R^{4a} bereits die gewünschte einen biolabilen Ester bildende Gruppe, z. B. die Ethylgruppe, darstellt und als Schutzgruppe R^{5a} in den Verbindungen der Formel III Schutzgruppen, welche unter Bedingungen gespalten werden, unter denen die R⁴-OCO-Gruppe nicht gespalten wird. Falls die R⁴-OCO-Gruppe die relativ säurestabile Ethylestergruppe ist, eignen sich als Schutzgruppe R^{5a} beispielsweise die durch Säure abspaltbare tert.-Butylgruppe oder eine hydrogenolytisch abspaltbare Gruppe wie Benzyl.

Falls R^{4a} in den Verbindungen der Formel II eine säureempfindliche einen biolabilen Ester bildende Gruppe darstellt, wird als Schutzgruppe R^{5a} in den Verbindungen der Formel III zweckmäßig eine hydrogenolytisch abspaltbare Gruppe wie Benzyl gewählt und diese aus den durch Umsetzung der Verbindungen der Formel II mit den Verbindungen der Formel III hervorgegangen Verbindungen der Formel IV hydrogenolytisch abgespalten. Die Hydrogenolyse kann durch katalytische Hydrierung in Gegenwart eines Katalysators, vorzugsweise eines Pd/C-Katalysators in einem unter den Reaktionsbedingungen inerten organischen Lösungsmittel, beispielsweise einem niederen Alkohol wie Ethanol oder einem niederen Alkylester wie Essigsäureethylester erfolgen. Zweckmäßig wird die katalytische Hydrierung bei einem Wasserstoffdruck von 4 bis 5 bar bei Raumtemperatur durchgeführt.

Zur Herstellung von Verbindungen der Formel I, worin R⁴ eine einen biolabilen Ester bildende Gruppe und R⁵ Wasserstoff bedeuten, können jedoch auch Ausgangsverbindungen der Formeln II und III mit unterschiedlichen Schutzgruppen R^{4a} und R^{5a} mit unterschiedlicher Reaktivität gewählt werden und aus den durch Umsetzung der Verbindungen der Formel II mit den Verbindungen der Formel III erhaltenen Verbindungen der Formel IV zunächst die Schutzgruppe R^{4a} unter Erhalt der Schutzgruppe R^{5a} abgespalten werden, dann in das Reaktionsprodukt der allgemeinen Formel IV'
(siehe Formel IV')
worin R¹, R², R³, R^{5a} und A obige Bedeutung besitzen, die gewünschte einen biolabilen Ester bildende Gruppe R⁴ durch Umsetzung der freien Säuregruppe der Verbindung der Formel IV' mit einer Verbindung der Formel V oder Va eingefügt werden und anschließend aus den erhaltenen Verbindungen der Formel IV die Schutzgruppe R^{5a} abgespalten werden.

So kann z. B. aus Verbindungen der Formel IV, worin R^{4a} eine durch Säure abspaltbare Schutzgruppe, insbesondere die tert.-Butylgruppe, und R^{5a} eine säurestabile Schutzgruppe, z. B. Benzyl, bedeuten zunächst nur die Schutzgruppe R^{4a} sauer abgespalten werden. Die erhaltene Monocarbonsäure der Formel IV' kann dann nach an sich zur Esterbildung üblichen Methoden mit einem Alkohol der Formel V oder einer entsprechenden Verbindung der Formel Va verestert werden. Als abspaltbare reaktive Gruppen X in den Verbindungen der Formel Va eignen sich Halogene, insbesondere Chlor oder Brom, oder ein organischer Sulfonsäurerest, beispielsweise der Rest einer Niederalkansulfonsäure wie z. B. Methansulfonsäure oder von aromatischen Sulfonsäuren wie Benzolsulfonsäure oder durch niederes Alkyl oder Halogen substituierte Benzolsulfonsäuren wie Toluolsulfonsäuren. Zur Veresterung können Alkohole der Formel V beispielsweise mit einer Säure der Formel IV' oder einem reaktiven Säurederivat dieser Säure auf an sich zur Acylierung von Alkoholen bekannte Weise umgesetzt werden. Die Umsetzung kann beispielsweise unter den für die Umsetzung von Verbindungen der Formel II mit Verbindungen der Formel III angegebenen Reaktionsbedingungen durchgeführt werden.

In analoger Weise können durch Auswahl entsprechender unterschiedlicher Schutzgruppen auch Verbindungen der Formel I hergestellt werden, worin R⁵ eine einen biolabilen Ester bildende Gruppe bedeutet und R⁴ für Wasserstoff oder eine von R⁵ unterschiedliche einen biolabilen Ester bildende Gruppe bedeutet.

Bei den vorgehend beschriebenen Umsetzungen werden die chiralen Zentren in den Ausgangsverbindungen der Formeln II und III nicht verändert, so daß je nach Art der Ausgangsverbindungen isomerenreine Verbindungen der Formel I oder Isomerengemische erhalten werden können. Zur Herstellung von isomerenreinen und somit optisch einheitlichen Verbindungen der Formel I werden zweckmäßigerweise enantiomerenreine Verbindungen der Formel II mit enantiomerenreinen Verbindungen der Formel III umgesetzt. Falls eine enantiomerenreine Verbindung der Formel II mit einer racemischen Verbindung der Formel III oder eine racemische Verbindung der Formel II mit einer enantiomerenreinen Verbindung der Formel III umgesetzt werden, wird jeweils ein Gemisch aus zwei Diastereomeren erhalten, welches gewünschtenfalls auf an sich bekannte Weise aufgetrennt werden kann. Die Umsetzung von racemischen Verbindungen der Formel II mit racemischen Verbindungen der Formel III ergibt entsprechende Gemische aus 4 Isomeren, welche gewünschtenfalls auf an sich bekannte Weise aufgetrennt werden können.

Die Ausgangsverbindungen der Formel II können nach an sich bekannten Methoden erhalten werden.

Beispielsweise können Verbindungen der allgemeinen Formel IIa
(siehe Formel IIa)
worin R^{4a} obige Bedeutung besitzt und R^{1a} die für R¹ angegebene Bedeutung mit Ausnahme eines Niederalkoxyniederalkoxymethylrestes besitzt, erhalten werden, indem man Acrylsäurederivate der allgemeinen Formel VI
(siehe Formel VI)
worin R^{4a} und R^{1a} obige Bedeutung besitzen mit Cyclopentancarbonsäure der Formel VII
(siehe Formel VII)
umsetzt. Die Reaktion kann auf an sich bekannte Weise unter den Bedingungen einer Michael-Addition in einem unter den Reaktionsbedingungen inerten organischen Lösungsmittel durch Reaktion der Cyclopentancarbonsäure mit einer starken, zur Bildung des Dianions der Cyclopentancarbonsäure befähigten, Base und anschließende Umsetzung mit dem Acrylesterderivat der Formel VI erfolgen. Als Lösungsmittel eignen sich Ether, insbesondere cyclische Ether wie beispielsweise Tetrahydrofuran. Als starke Basen eignen sich nicht-nucleophile, organische Alkalimetallamide wie z. B. Lithiumdiisopropylamid. Zweckmäßig wird die Cyclopentancarbonsäure in Tetrahydrofuran mit zwei Äquivalenten Lithiumdiisopropylamid umgesetzt und das Reaktionsgemisch anschließend mit der Verbindung der Formel VI weiter umgesetzt. Die Reaktionstemperatur kann zwischen -70 und 0 °C betragen.

Verbindungen der allgemeinen Formel IIb
(siehe Formel IIb)
worin R^{4a} obige Bedeutung besitzt und R^{1b} einen Niederalkoxyniederalkoxymethylrest bedeutet, können erhalten werden, indem man Halogencarbonsäureester der allgemeinen Formel VIII
(siehe Formel VIII)
worin R^{4a} obige Bedeutung besitzt und Y Halogen bedeutet, mit Cyclopentancarbonsäure der Formel VII umsetzt und das erhaltene Reaktionsprodukt der allgemeinen Formel IX
(siehe Formel IX)
worin R^{4a} obige Bedeutung besitzt, mit Verbindungen der allgemeinen Formel Xb
(siehe Formel Xb)
worin R^{1b} und X obige Bedeutung besitzten, umsetzt. Die Umsetzung der Halogencarbonsäureester der Formel VIII mit der Cyclopentancarbonsäure der Formel VII kann auf an sich bekannte Weise in einem unter den Reaktionsbedingungen inerten Lösungsmittel in Gegenwart einer starken zur Bildung des Dianions der Cyclopentancarbonsäure befähigten Base erfolgen. Beispielsweise kann die Umsetzung unter den für die Umsetzung von Cyclopentancarbonsäure mit Verbindungen der Formel VI angegebenen Bedingungen durchgeführt werden. Die anschließende Umsetzung der Säuren der Formel IX mit Verbindungen der Formel Xb kann auf an sich bekannte Weise unter zur α-Alkylierung von Carbonsäureestern geeigneten Bedingungen in einem unter den Reaktionsbedingungen inerten organischen Lösungsmittel in Gegenwart einer starken Base durchgeführt werden. Vorzugsweise werden Verbindungen der Formel Xb eingesetzt, in denen X Chlor oder Brom bedeutet. Als Lösungsmittel eignen sich Ether, insbesondere cyclische Ether wie Tetrahydrofuran oder Dioxan. Als starke Base können Alkalimetallhydride oder -amide beispielsweise Lithiumdiisopropylamid eingesetzt werden.

Die Verbindungen der Formel II besitzen an dem den Rest R¹ tragenden Kohlenstoffatom ein Chiralitätszentrum und werden bei der Synthese in Form ihrer Racemate erhalten. Die optisch aktiven Verbindungen können aus den racemischen Gemischen in an sich bekannter Weise erhalten werden, z. B. durch chromatographische Trennung an chiralen Trennmaterialien oder durch Umsetzung mit geeigneten optisch aktiven Basen, z. B. α-Methylbenzylamin oder Pseudoephedrin, und anschließende Auftrennung in ihre optischen Antipoden durch fraktionierte Kristallisation der gewonnen Salze.

Acrylsäureesterderivate der Formel VI können auf an sich bekannte Weise erhalten werden, indem man (Diniederalkylphosphono)-essigsäureester-Derivate der allgemeinen Formel XI
(siehe Formel XI)
worin R^{4a} und R^{1a} obige Bedeutung besitzen und R⁷ und R⁸ je niederes Alkyl, vorzugsweise Methyl oder Ethyl, bedeuten, mit Formaldehyd in einem unter den Reaktionsbedingungen inerten organischen Lösungsmittel unter basischen Bedingungen umsetzt. Beispielsweise können Verbindungen der Formel XI mit Paraformaldehyd in einem Ether, vorzugsweise einem cyclischen Ether wie Tetrahydrofuran, in Gegenwart einer Base, vorzugsweise eines nicht-nukleophilen Alkalimetallalkoholates wie Kalium-tert.-butylat bei Temperaturen zwischen -20 und +30 °C umgesetzt werden.

Verbindungen der Formel XI können auf an sich bekannte Weise erhalten werden, indem man Phosphonoessigsäure-Derivate der allgemeinen Formel XII
(siehe Formel XII)
worin R^{4a}, R⁷ und R⁸ obige Bedeutung besitzen, mit Verbindungen der Formel Xa
(siehe Formel Xa)
worin R^{a} und X obige Bedeutung besitzen, umsetzt. Die Umsetzung kann unter zur Alkylierung üblichen Bedingungen in einem unter den Reaktionsbedingungen inerten polaren aprotischen organischen Lösungsmittel in Gegenwart einer Base bei Temperaturen zwischen 0 und 80 °C durchgeführt werden. Vorzugsweise werden Verbindungen der Formel Xa eingesetzt, worin X Halogen, insbesondere Brom oder Jod, oder Tosylat bedeutet. Als Lösungsmittel eignen sich beispielsweise Amide wie Dimethylformamid oder auch Ether. Als Basen eignen sich nicht-nukleophile Alkalimetallalkoholate wie beispielsweise Kalium-tert.-butylat.

Verbindungen der Formel VI können auch erhalten werden, indem man Malonsäurederivate der allgemeinen Formel XIII
(siehe Formel XIII),
worin R^{4a} und R^{1a} obige Bedeutung besitzen, auf an sich bekannte Weise mit Formaldehyd unter basischen Bedingungen behandelt. So können Malonsäurederivate der Formel XIII beispielsweise mit wäßriger Formaldehydlösung in Gegenwart eines sekundären organischen Amins, insbesondere Piperidin, bei Temperaturen zwischen 0 und 30 °C, vorzugsweise bei unterhalb Raumtemperatur liegenden Temperaturen, umgesetzt werden. Die Malonsäure-Derivate der Formel XIII können auch mit Paraformaldehyd in Pyridin bei Temperaturen zwischen 40 bis 60 °C umgesetzt werden.

Die Malonsäuremonoester der Formel XIII können erhalten werden, indem man Malonsäurediester der allgemeinen Formel XIV
(siehe Formel XIV)
worin R^{4a} obige Bedeutung besitzt und R⁹ niederes Alkyl, insbesondere Methyl, oder Benzyl bedeutet, mit Verbindungen der Formel Xa umsetzt und die erhaltenen Malonsäurediester-Derivate der allgemeinen Formel XV
(siehe Formel XV)
worin R^{1a}, R^{4a} und R⁹ obige Bedeutung besitzen, durch partielle Hydrolyse in die entsprechenden Malonsäuremonoester-Derivate der Formel XIII überführt.

Die Einführung des Restes R^{1a} in die Malonsäurediester der Formel XIV kann auf an sich bekannte Weise durch Umsetzung der Ester der Formel XIV mit einer Verbindung der Formel Xa in einem polaren aprotischen organischen Lösungsmittel, vorzugsweise Dimethylformamid, in Gegenwart einer Base, beispielsweise einem nicht-nukleophilen Alkalimetall-Alkoholat wie Kaliumtert.-butylat bei Temperaturen zwischen 0 °C und 80 °C erfolgen. Die Umsetzung kann beispielsweise unter den für die Umsetzung von Verbindungen der Formel XI mit Verbindungen der Formel Xa angegebenen Bedingungen durchgeführt werden.

Die erhaltenen substituierten Malonsäurediester der Formel XV können durch Abspaltung des Restes R⁹ auf an sich bekannte Weise in die entsprechenden Malonsäuremonoester der Formel XIII überführt werden. Sofern die Schutzgruppe R^{4a} und der Rest R⁹ verschiedene Reste mit unterschiedlicher Reaktivität darstellen, werden für die Abspaltung des Restes R⁹ zweckmäßigerweise solche Bedingungen gewählt, unter denen der Rest R^{4a} nicht angegriffen wird. Sofern R⁹ Benzyl bedeutet, kann die Abspaltung auf an sich bekannte Weise hydrogenolytisch erfolgen. Niedere Alkylester R⁹ werden hydrolytisch, je nach Art des Alkylrestes unter sauren oder alkalischen Bedingungen auf an sich bekannte Weise abgespalten. Vorzugsweise stellt R⁹ Ethyl dar, welches durch alkalische Hydrolyse abgespalten werden kann. Hierzu können die Alkylester der Formel XV in einem niederen Alkohol oder einem Gemisch aus einem niederen Alkohol mit Wasser mit einem Alkalimetallhydroxid, beispielsweise Kaliumhydroxid, behandelt werden. Sofern die Reste R^{4a} und R⁹ identisch sind, wird hierbei die Menge an Alkalimetallhydroxid so gering gehalten, daß nur eine partielle Hydrolyse eintritt.

Verbindungen der Formel III können auf an sich bekannte Weise erhalten werden, indem man Verbindungen der allgemeinen Formel XVI
(siehe Formel XVI)
worin R², R³ und A obige Bedeutung besitzen und die R¹⁰R¹¹N-Gruppe eine durch eine Aminoschutzgruppe geschützte Aminogruppe darstellt, mit Verbindungen der allgemeinen Formel XVII
(siehe Formel XVII)
worin R^{5a} und X obige Bedeutung besitzen, umsetzt und in dem erhaltenen Reaktionsprodukt der allgemeinen Formel XVIII
(siehe Formel XVIII)
worin R², R³, R^{5a}, A und die R¹⁰R¹¹N-Gruppe obige Bedeutung besitzen, aus der R¹⁰R¹¹N-Gruppe die freie Aminogruppe freisetzt. Die Umsetzung von Verbindungen der Formel XVI mit Verbindungen der Formel XVII kann nach an sich zur Alkylierung von Amiden üblichen Methoden durchgeführt werden. Vorzugsweise werden Verbindungen der Formel XVII eingesetzt, in welchen X für Halogen, vorzugsweise Brom oder Jod, steht. Die Umsetzung kann in einem polaren aprotischen organischen Lösungsmittel, beispielsweise Dimethylformamid oder einem cyclischen Ether wie Tetrahydrofuran in Gegenwart einer Base durchgeführt werden. Als Basen eignen sich nicht-nukleophile Basen wie beispielsweise Kalium-tert.-butylat. Gewünschtenfalls kann die Umsetzung auch in Gegenwart eines Alkalimetallhydroxides, z. B. Kaliumhydroxid, in einem Zweiphasensystem in Gegenwart eines Phasentransferkatalysators, beispielsweise eines Tetraniederalkylammoniumhalogenides wie Tetrabutylammoniumbromid, durchgeführt werden.

Anschließend kann in den erhaltenen Verbindungen der Formel XVIII die Aminogruppe durch Abspaltung der Schutzgruppe in an sich bekannter Weise freigesetzt werden. Zum Schutz der Aminogruppe können die an sich zum Schutze von Aminogruppen bekannten leicht wieder abspaltbaren Schutzgruppen, beispielsweise die aus der Peptidchemie bekannten Schutzgruppen, eingesetzt werden. Geeignete Schutzgruppen sind z. B. bekannt aus E. McOmie "protective groups in organic chemistry" Plenum Press 1971. Beispielsweise eignen sich als Schutzgruppen die Phthalimidgruppe oder die tert.-Butoxycarbonylgruppe oder auch die Benzyloxycarbonylgruppe. Es müssen in Abhängigkeit der Bedeutung von R^{5a} jeweils Schutzgruppen gewählt werden, die anschließend unter Bedingungen abspaltbar sind, unter denen die Gruppe R^{5a} nicht angegriffen wird. Als in basischem Medium abspaltbare Schutzgruppe eignet sich beispielsweise die Phthalimidgruppe, welche durch Behandeln mit Ethanolamin oder mit Hydrazin bei erhöhten Temperaturen, beispielsweise Temperaturen zwischen 70 und 90 °C, abgespalten werden kann. Die Phthalimidgruppe eignet sich beispielsweise als Schutzgruppe für Verbindungen, worin A Schwefel ist. Als sauer abspaltbare Schutzgruppe eignet sich beispielsweise die tert.-Butoxycarbonyl-Gruppe, welche durch Behandlung mit Säure, beispielsweise durch Behandeln mit Trifluoressigsäure oder mit Chlorwasserstoffgas in Essigsäureethylester, wieder abgespalten werden kann. Die tert.-Butoxycarbonyl-Gruppe eignet sich z. B. als Schutzgruppe für Verbindungen worin A Sauerstoff ist. Als hydrogenolytisch abspaltbare Schutzgruppe eignet sich beispielsweise die Benzyloxycarbonyl-Gruppe, welche durch Hydrierung mit Wasserstoff in Gegenwart eines Palladium/Kohle-Katalysators abgespalten werden kann.

Die Verbindungen der Formel III enthalten an dem die Aminogruppe tragenden Kohlenstoffatom ein Chiralitätszentrum. Sofern von optisch reinen Ausgangsverbindungen der Formel XVI ausgegangen wird, werden optisch reine Verbindungen der Formel III erhalten. Dies trifft insbesonders für solche Verbindungen zu, worin A Sauerstoff oder Schwefel bedeutet. Sofern von racemischen Verbindungen der Formel XVI ausgegangen wird, werden auch racemische Verbindungen der Formel III erhalten. Dies ist im allgemeinen bei Verbindungen, worin A eine Methylengruppe bedeutet, der Fall. Racemische Gemische von Verbindungen der Formel III können auf an sich bekannte Weise in ihre optischen Isomeren aufgetrennt werden, beispielsweise durch chromatographische Trennung an chiralen Trennmaterialien oder durch Umsetzung mit geeigneten optisch aktiven Säuren, beispielsweise Weinsäure, und anschließende Auftrennung der optischen Antipoden durch fraktionierte Kristallisation der gewonnenen Salze. Zur Erhöhung der Ausbeute an dem gewünschten optischen Isomer kann bei der Umsetzung mit geeigneten optisch aktiven Säuren gleichzeitig mit oder nach der weitgehenden Fällung des Salzes des einen Isomeren mit der optisch aktiven Säure in dem Reaktionsgemisch eine Re-Racemisierung des in Lösung verbliebenen Isomeren durch Zusatz eines vorzugsweise aromatischen Aldehydes wie beispielsweise Benzaldehyd in Gang gesetzt werden. Dabei wird die Racemisierung an dem Chiralitätszentrum durch Iminbildung mit dem Aldehyd hervorgerufen.

Die Verbindungen der Formel XVI können auf an sich bekannte Weise erhalten werden. Beispielsweise können Verbindungen der allgemeinen Formel XVIa
(siehe Formel XVIa)
worin R², R³ und die R¹⁰R¹¹N-Gruppe obige Bedeutung besitzen, erhalten werden, indem man in Verbindungen der allgemeinen Formel XIX
(siehe Formel XIX)
worin R², R³ und Y obige Bedeutung besitzen, das Halogen Y durch die R¹⁰R¹¹N-Gruppe auf an sich bekannte Weise ersetzt. Beispielsweise kann eine Verbindung der Formel XIX mit einem Alkalimetallsalz eines Amides R¹⁰R¹¹NH, vorzugsweise KaliumPhthalimid, umgesetzt werden. Die Umsetzung kann in einem unter den Reaktionsbedingungen inerten aprotischen organischen Lösungsmittel, vorzugsweise Dimethylformamid, bei Temperaturen zwischen 40 und 80 °C erfolgen.

Verbindungen der Formel XIX können auf an sich bekannte Weise durch Beckmann-Umlagerung von Oxim-Verbindungen der allgemeinen Formel XX
(siehe Formel XX)
worin R², R³ und Y obige Bedeutung besitzen, erhalten werden, indem man Verbindungen der Formel XX unter Bedingungen einer Beckmann-Umlagerung mit einer Säure behandelt. Zweckmäßigerweise werden Verbindungen der Formel XX durch Behandeln mit Polyphosphorsäure bei Temperaturen zwischen 60 und 90 °C in die Verbindungen der Formel XIX umgelagert.

Oxime der Formel XX können ausgehend von cyclischen Ketonen der allgemeinen Formel XXI
(siehe Formel XXI)
worin R² und R³ obige Bedeutung besitzen, erhalten werden, indem man die Ketone der Formel XXI zunächst zur Einführung des Restes Y mit Halogen behandelt und die erhaltenen halogenierten Ketone anschließend mit Hydroxylamin umsetzt. Zweckmäßigerweise können die α-Halogenierung des Ketones und die anschließende Oximbildung in einem Eintopfverfahren durchgeführt werden, wobei das Keton der Formel XXI zunächst in einem inerten organischen Lösungsmittel, beispielsweise einem niederen Alkohol wie Methanol, mit dem Halogen behandelt wird und anschließend dem Reaktionsgemisch Hydroxylamin zugeführt wird. Zweckmäßigerweise wird das Hydroxylamin in Form eines Hydroxylamin-Salzes, beispielsweise des Hydrochlorides, eingesetzt und dem Reaktionsgemisch etwas Wasser zugefügt. Das Verfahren kann bei Temperaturen zwischen 0 und 40 °C, vorzugsweise bei Raumtemperatur, durchgeführt werden.

Verbindungen der allgemeinen Formel XVIb
(siehe Formel XVIb)
worin R², R³ und die R¹⁰R¹¹N-Gruppe obige Bedeutung besitzen und A^{a} für Sauerstoff oder Schwefel steht, können auf an sich bekannte Weise durch Cyclisierung von aromatischen Aminosäureverbindungen der allgemeinen Formel XXII
(siehe Formel XXII)
worin R², R³, A^{a} und die R¹⁰R¹¹N-Gruppe obige Bedeutung besitzen, erhalten werden. Die Cyclisierung der Verbindungen der Formel XXII verläuft unter Wasserabspaltung und kann nach an sich zur Lactambildung üblichen Methoden erfolgen. So kann die Cyclisierung beispielsweise in Gegenwart eines die Säuregruppe aktivierenden aus der Peptidchemie zur Amidbildung bekannten Kopplungsreagenzes, beispielsweise eines Carbodiimids, in einem unter den Reaktionsbedingungen inerten polaren organischen Lösungsmittel, beispielsweise Dimethylformamid, erfolgen. Die Umsetzung kann beispielsweise unter den für die Umsetzung von Verbindungen der Formel II mit Verbindungen der Formel III angegebenen Bedingungen durchgeführt werden. Als die Säuregruppe aktivierendes Agens kann auch Diethylphosphonocyanid eingesetzt und die Umsetzung in Gegenwart einer organischen Base, beispielsweise eines Triniederalkylamins wie Triethylamin, durchgeführt werden.

Verbindungen der allgemeinen Formel XXII können auf an sich bekannte Weise durch Reduktion entsprechender Nitroverbindungen der allgemeinen Formel XXIII
(siehe Formel XXIII)
worin R², R³, A^{a} und die R¹⁰R¹¹N-Gruppe obige Bedeutung besitzen, erhalten werden. Die Reduktion der Nitrogruppe kann nach an sich zur Reduktion von Nitrobenzolverbindungen zu Anilinverbindungen bekannten Methoden beispielsweise durch katalytische Hydrierung in Gegenwart eines Palladium/Kohle-Katalysators durchgeführt werden. Die Reduktion kann auch unter Verwendung anderer Reduktionsmittel, welche Wasserstoff in situ erzeugen, beispielsweise metallisches Eisen/Salzsäure oder metallisches Zink/Salzsäure, durchgeführt werden.

Verbindungen der Formel XXIII können auf an sich bekannte Weise durch Umsetzung von o-Fluornitrobenzol-Verbindungen der allgemeinen Formel XXIV
(siehe Formel XXIV)
worin R² und R³ obige Bedeutung besitzen, mit Säuren der allgemeinen Formel XXV
(siehe Formel XXV)
worin A^{a} und die R¹⁰R¹¹N-Gruppe obige Bedeutung besitzen erhalten werden. Die Verbindungen der Formel XXV stellen Serin- bzw. Cysteinderivate, deren Aminogruppe geschützt ist, dar. Die Umsetzung erfolgt in einem unter den Reaktionsbedingungen inerten organischen Lösungsmittel in Gegenwart einer Base. Die Umsetzung der Fluornitrobenzole mit dem stark nukleophilen Cysteinderivat kann in einem niederen Alkohol oder einem Alkohol/Wassergemisch in Gegenwart einer schwachen Base wie Natriumhydrogencarbonat durchgeführt werden. Zur Umsetzung mit dem vergleichsweise schwächer nukleophilen Serinderivat wird zweckmäßigerweise eine starke Base, beispielsweise ein Alkalimetallhydrid, in einem polaren organischen Lösungsmittel wie Dimethylformamid verwendet.

Gewünschtenfalls kann nach Bildung der Verbindungen der Formel XXIII die ursprünglich in den Verbindungen der Formel XXV vorhandene Aminoschutzgruppe auf an sich bekannte Weise gegen eine andere Aminoschutzgruppe ausgetauscht werden, welche sich in ihrer Reaktivität von dem Rest R^{5a} besser unterscheidet und somit besser für die Weiterverarbeitung der Verbindungen der Formel XXIII geeignet ist.

Die Erfindung betrifft auch Arzneimittel enthallend eine pharmakologisch wirksame Menge einer Verbindung gemäss Formel I.

Die Verbindungen der Formel I und ihre pharmakologisch akzeptablen Salze zeichnen sich durch interessante pharmakologische Eigenschaften aus. Insbesondere üben die Substanzen eine inhibitorische Wirkung auf die neutrale Endopeptidase (= NEP) aus. NEP ist ein Enzym, welches den Abbau endogener natriuretischer Peptide, z. B. des atrialen natriuretischen Peptids (= ANP), bewirkt. Durch ihre Hemmwirkung auf die NEP-Aktivität vermögen die Substanzen die biologische Aktivität und Lebensdauer der durch NEP angreifbaren natriuretischen Peptide, insbesondere des ANP, zu verbessern und eignen sich deshalb für die Behandlung von durch die Wirkung derartiger Hormone günstig beeinflußten Krankheitszuständen, insbesondere Herzinsuffizienz.

Bei Herzinsuffizienz kommt es durch die krankheitsbedingte reduzierte kardiale Auswurfleistung des Herzens zu einem reflektorisch erhöhten peripheren Widerstand und damit zu einem Rückstau des Blutes in den Lungenkreislauf und das Herz selbst. Es entsteht in der Folge ein hoher Herzfüllungsdruck, der eine Kammerwanddehnung in den Herzvorhöfen und den Herzkammern verursacht. Unter diesen Umständen funktioniert das Herz wie ein endokrines Organ, d. h es ist in der Lage, ANP, welches ausgeprägte vasodilatorische und diuretisch/natriuretische Aktivitäten besitzt, in die Blutbahn zu sezernieren. ANP wirkt reduzierend auf den erhöhten Herzfüllungsdruck. Dieses geschieht durch Diurese/Natriurese (Reduktion des zirkulierenden Blutvolumens) und durch Reduktion des peripheren Widerstandes (Vor- und Nach- lastsenkung). Die herzentlastende Wirkung von ANP wird als endogener kardioprotektiver Mechanismus angesehen. Die Wirkung von ANP ist aber nur kurzzeitig, da das Hormon von NEP rasch gespalten wird.

Auf Grund ihrer NEP-hemmenden Eigenschaften vermögen die erfindungsgemäßen Verbindungen den kardioprotektiven Wirkmechanismus des ANP zu verbessern und weisen insbesondere eine hohe Effektivität bezüglich einer Verstärkung diuretisch/natriuretischen Aktivitäten auf.

Die erfindungsgemäßen Verbindungen zeichnen sich durch ein günstiges Wirkprofil mit guter Verträglichkeit aus und weisen dabei eine weitgehende Selektivität der NEP-inhibitorischen Wirkung auf und lassen zusätzlich noch geringe inhibitorische Wirkungen auf das Endothelin convertierende Enzym (= ECE) erkennen. Bei höhergradigen Stadien der Herzinsuffizierung kommt es reflektorisch zu hohen Blutspiegeln an Angiotensin II, Endothelin und Katecholaminen und damit zu weiterer Erhöhung des peripheren Widerstandes und des Herzfüllungsdruckes, wodurch der Herzmuskel hypertrophiert und dilatiert. Die ECE-inhibitorischen Zusatzeigenschaften können hierbei die reduzierende Wirkung der erfindungsgemäßen Substanzen auf den peripheren Widerstand verstärken.

Die NEP- und ECE-inhibitorischen und die Diurese/Natriurese verstärkenden Eigenschaften der Substanzen wurden in pharmakologischen Standardtestmethoden in vitro und in vivo nachgewiesen.

Beschreibung der pharmakologischen Untersuchungsmethoden:

### 1. Bestimmung der minimalen toxischen Dosis

Männlichen Mäusen von 20 - 25 g Gewicht werden p. o. Maximaldosen von 300 mg/kg der Testsubstanz verabreicht. Die Tiere werden 3 Stunden lang sorgfältig auf Toxizitätssymptome beobachtet. Über einen Zeitraum von 72 Stunden nach der Applikation werden zusätzlich alle Symptome und Todesfälle registriert. Begleitsymptome werden ebenfalls beobachtet und registriert. Wenn Tod oder starke toxische Symptome beobachtet werden, werden weiteren Mäusen zunehmend geringere Dosen verabreicht, bis keine toxischen Symptome mehr auftreten. Die niedrigste Dosis, welche Tod oder starke toxische Symptome hervorruft, wird in der nachfolgenden Tabelle A als minimale toxische Dosis angegeben. Die in Tabelle A angegebenen Beispielnummern beziehen sich auf die nachstehenden Herstellungsbeispiele.

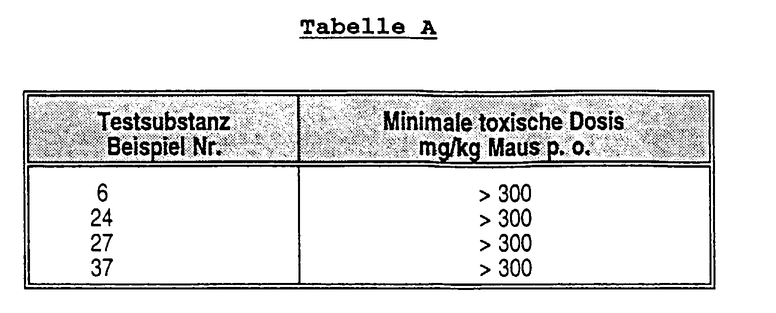

### 2. In vitro Untersuchung der NEP-Hemmwirkung der Substanzen und Bestimmung der Affinität der Substanzmoleküle zu dem Enzymmolekül

Zum Nachweis für die Hemmwirkung der erfindungsgemäßen Substanzen auf die neutrale Endopeptidase (= NEP) wurde in einem Standardtest in vitro die inhibitorische Wirkung der Substanzen auf den durch die enzymatische Aktivität der NEP stattfindenden hydrolytischen Abbau von Methionin-Enkephalin (= Met-Enkephalin) untersucht. Dabei wurde als Maß für die inhibitorische Wirksamkeit der Substanzen deren Ki-Wert (= Inhibitorkonstante) bestimmt. Der Ki-Wert einer enzyminhibierend wirksamen Testsubstanz ist die Dissoziationskonstante des Enzym-Testsubstanz-Komplexes bzw. des (Enzym-Substrat)-Testsubstanz-Komplexes und hat die Dimension einer Konzentration.

### Testdurchführung

Zur Testdurchführung werden jeweils 100 µl Proben verschiedener Inkubationslösungen enthaltend 10 ng gereinigte NEP (E.C.3.4.24.11) und jeweils unterschiedliche Mengen an Testsubstanz und an Substrat (Met-Enkephalin) und 50 mM Tris-puffer (= Tris(hydroxymethyl)aminomethan/HCl, pH 7,4) hergestellt.

Pro Testsubstanz werden jeweils 24 verschiedene Inkubationslösungen hergestellt mit 3 verschiedenen Testsubstanzkonzentrationen jeweils in Kombination mit Met-Enkephalin-Gehalten von 2, 5, 7, 10, 12, 15, 40 und 100 µM.

Bei jedem Test werden jeweils auch zweierlei Kontroll-Inkubationslösungen mitbehandelt, zum einen Enzymkontrollen, welche keine Testsubstanz enthalten, und zum anderen Substratkontrollen, welche weder Enzym noch Testsubstanz enthalten.

Die Inkubationslösungen werden für 45 Minuten bei 37 °C im Schüttel-Wasserbad inkubiert. Dabei wird die Enzymreaktion nach 15 Minuten durch Zugabe des Substrates (Met-Enkephalin) gestartet und am Ende der Inkubationszeit durch Erhitzen für 5 Minuten bei 95 °C gestoppt. Anschließend wird die gestoppte Inkubationslösung für 3 Minuten bei 12.000 x g zentrifugiert, und in dem Überstand werden die Konzentrationen an nicht umgesetztem Substrat und an durch die enzymatische Reaktion gebildeten Hydrolyseprodukten bestimmt. Hierzu werden jeweils Proben der Überstände durch HPLC (= Hochdruckflüssigchromatographie) an hydrophobisiertem Kieselgel aufgetrennt, und die Produkte der enzymatischen Reaktion und nicht umgesetztes Substrat werden photometrisch bei einer Wellenlänge von 205 nm bestimmt. Für die HPLC-Trennung wird eine Trennsäule (4,6 x 250 mm), welche als Reversed-Phase-Trennmaterial Encapharm® 100 RP18, 5 µ beinhaltet, eingesetzt. Der Lösungsmittelfluß beträgt 1,4 ml/min, die Säule wird auf 40 °C erwärmt. Fließmittel A ist 5 mM H₃PO₄, pH 2,5 und Fließmittel B ist Acetonitril + 1 % 5 mM H₃PO₄, pH 2,5.

Aus den in den verschiedenen Proben gemessenen Konzentrationen an Hydrolyseprodukten und nicht umgesetztem Substrat wird für jede Testsubstanz der Ki-Wert auf an sich bekannte Weise berechnet. Die nachstehende Tabelle B gibt die für die Testsubstanzen gefundenen Ki-Werte wieder. Die in Tabelle B angegebenen Beispielsnummern beziehen sich auf die nachstehenden Herstellungsbeispiele.

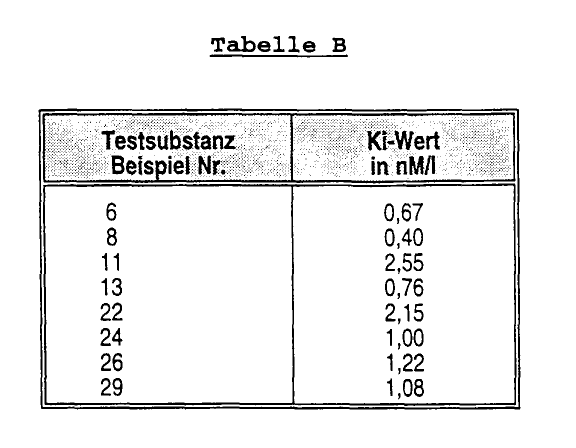

### 3. In vitro Untersuchungen der ECE-Hemmwirkung der Substanzen

Zum Nachweis für die Hemmwirkung der erfindungsgemäßen Substanzen auf das Endothelin convertierende Enzym (= ECE) wurde in einem Standardtest in vitro die inhibitorische Wirkung der Substanzen auf den durch die enzymatische Aktivität des ECE stattfindenden hydrolytischen Abbau von Big-Endothelin 1 (bigET-1) untersucht. Dabei wurde als Maß für die inhibitorische Wirksamkeit der Substanzen deren IC₅₀-Wert bestimmt. Der IC₅₀-Wert einer enzyminhibierend wirksamen Testsubstanz ist die Konzentration der Testsubstanz bei der 50 % der enzymatischen Aktivität der ECE inhibiert sind.

### Herstellung der das ECE enthaltenden Endothelzell-Membranfraktion

Eizellen des chinesischen Hamsters (= Chinese hamster ovarian cells, im folgenden abgekürzt als CHO-Zellen), in denen humanes ECE rekombinant exprimiert wurde (siehe Schmidt et al., Federation of European Biochemical Societies Letters 356 (1994), 238 bis 243), werden lysiert und die Zellmembranen durch Zentrifugation bei 10000 x g für 10 min abgetrennt. Durch 3-maliges Resuspendieren und wiederholtes Zentrifugieren werden die Zellmembranen gewaschen. Die ECE enthaltende Zellmembranfraktion wird in 100 mM Tris/HCL-Puffer (= Tris(hydroxymethyl)aminomethan/HCl, pH 7,0 enthaltend 250 mM NaCl) resuspendiert und bis zum Enzymtest bei -70 °C eingefroren aufbewahrt.

### Testdurchführung

Zur Testdurchführung werden jeweils 100 µl Proben verschiedener Inkubationslösungen enthaltend 5 µg Protein einer ECE enthaltenden Präparation von Endothelzellmembranen und jeweils unterschiedliche Mengen an Testsubstanz und 24 µM Substrat (synthetisches Peptid: H2N-Asp-Ile-Ala-Trp-Phe-Asn-Thr-Pro-Glu-His-Val-Val-Pro-Tyr-Gly-Leu-Gly-COOH) und 100 mM Tris-puffer (=Tris(hydroxymethyl)aminomethan/HCl, pH 7,0 enthaltend 250 mM Natriumchlorid hergestellt. Darüber hinaus sind in jeder Inkubationslösung 100 µM Thiorphan und 10 µM Captopril und 1 mM Phenylsulfonylfluorid, 100 µM Pepstatin A (= Proteaseinhibitor) und 100 µM Amastatin (= Proteaseinhibitor) enthalten.

Pro Testsubstanz werden jeweils 6 verschiedene Inkubationslösungen hergestellt mit 3 verschiedenen Testsubstanzkonzentrationen jeweils als Doppelbestimmungen.

Bei jedem Test wird jeweils auch eine Kontrolle mitbehandelt, welche kein Enzym enthält.

Die Inkubationslösungen werden für 15 min bei 37 °C vorinkubiert, bevor das Substrat zugegeben wird. Die Enzymreaktion wird durch Zugabe des Substrates gestartet; sie dauert 60 min, erfolgt bei 37 °C und wird durch Erhitzen der Inkubationslösungen für 5 min auf 95 °C gestoppt. Die durch die enzymatische Reaktion aus dem Substrat gebildeten Hydrolyseprodukte H₂N-Asp-Ile-Ala-Trp-COOH und H₂N-Phe-Asn-Thr-Pro-Glu-His-Val-Val-Pro-Tyr-Gly-Leu-Gly-COOH werden mit Hilfe der Hochdruck-Flüssigkeitschromatographie (HPLC) bestimmt. Die Durchführung der HPLC-Bestimmung erfolgt wie im Falle der vorgehend beschriebenen in vitro Untersuchung der NEP-Hemmwirkung.

Aus den in den verschiedenen Proben gemessenen Konzentrationen an Hydrolyseprodukten wird für jede Testsubstanz die IC₅₀ auf an sich bekannte Weise berechnet. Die nachstehende Tabelle C gibt die für die Testsubstanzen gefundenen IC₅₀-Werte wieder.

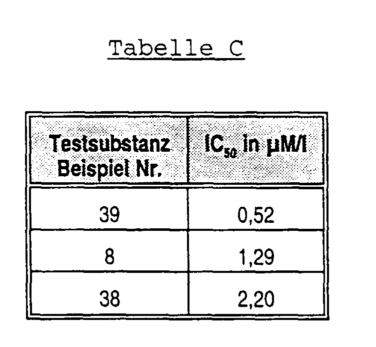

### 4. In vivo Bestimmung des Einfluß der Substanzen auf Diurese/ Natriurese in volumenbelasteten Ratten.

Die in vivo Aktivität wurde in der volumenbelasteten Ratte untersucht. In diesem Experiment wird durch eine Infusion von isotonischer Natriumchloridlösung ein hoher kardialer Füllungsdruck verursacht, in dessen Folge es zu einer ANP-Freisetzung und dadurch zu einer Diurese/Natriurese kommt.

### Testdurchführung:

Die Versuche werden mit männlichen Wistar Ratten mit einem Körpergewicht von 200 - 400 g durchgeführt. Unter Neurolept-Analgesie (Fentanyl; Hypnorm®, Hersteller Fa. Janssen) wird ein Katheter in die rechte Femoralvene eingebunden für die Hintergrundinfusion und die Volumenbelastung mit isotonischer Natriumchloridlösung. Nach Eröffnung der Bauchhöhle wird ein zweiter Katheter in die Blase eingesetzt und die Harnröhre abgebunden, so daß eine Messung von Harnvolumen, Natriurese und Kaliurese möglich wird.

Der Bauchraum wird wieder verschlossen und die Tiere bekommen eine Dauerinfusion mit Natriumchloridlösung (0,5 ml/ 100 g Körpergewicht) über den gesamten Versuchszeitraum von 2 Stunden. Nach einer Äquilibrierungsperiode von 30 Minuten werden in einer Vorlaufphase vor der Testsubstanzgabe dreimal jeweils über einen Zeitraum von 10 Minuten Urinproben gesammelt. Diese Vorwerte (= "Predrug"-Werte) werden bestimmt um zu überprüfen, daß bei den Testtieren ein kontinuierlicher Urinfluß stattfindet.

Sodann werden die Testsubstanzen enthaltende Lösungen intravenös (Bolus-Injektion in die Femoralvene) oder oral (mittels Schlundsonde) an Gruppen von jeweils 10 Ratten verabreicht. Für beide Applikationsformen erhält jeweils eine Kontrolltiergruppe lediglich Placebo-Lösungen, die keinen Wirkstoff enthalten. 5 Minuten nach i.v. Applikation bzw. 120 Minuten nach oraler Gabe der Substanzen werden die Ratten mit einem erhöhten Volumen Natriumchloridlösung i.v. belastet (2 ml/100 g Körpergewicht in 2 Minuten) und der Urin wird über einen Zeitraum von 60 min. gesammelt. Die in diesem Zeitraum anfallenden Urinmengen werden bestimmt und die darin enthaltenen Natrium- und Kaliumgehalte gemessen. Aus den angefallenen Urinmengen wird die unter der Volumenbelastung erfolgte Steigerung der Ausscheidung gegenüber den Vorlaufwerten abgelesen.

In der nachfolgenden Tabelle D werden die unter Volumenbelastung nach Testsubstanzgabe aufgetretenen Steigerungen der Harnausscheidung in % bezogen auf die unter Volumenbelastung nach Placebogabe erfolgten Steigerungen der Harnausscheidung angegeben. Ferner werden auch die unter Volumenbelastung nach Testsubstanzgabe ausgeschiedenen Mengen an Natrium und Kalium in % der unter Volumenbelastung nach Placebogabe ausgeschiedenen Natrium- und Kaliummengen angegeben.

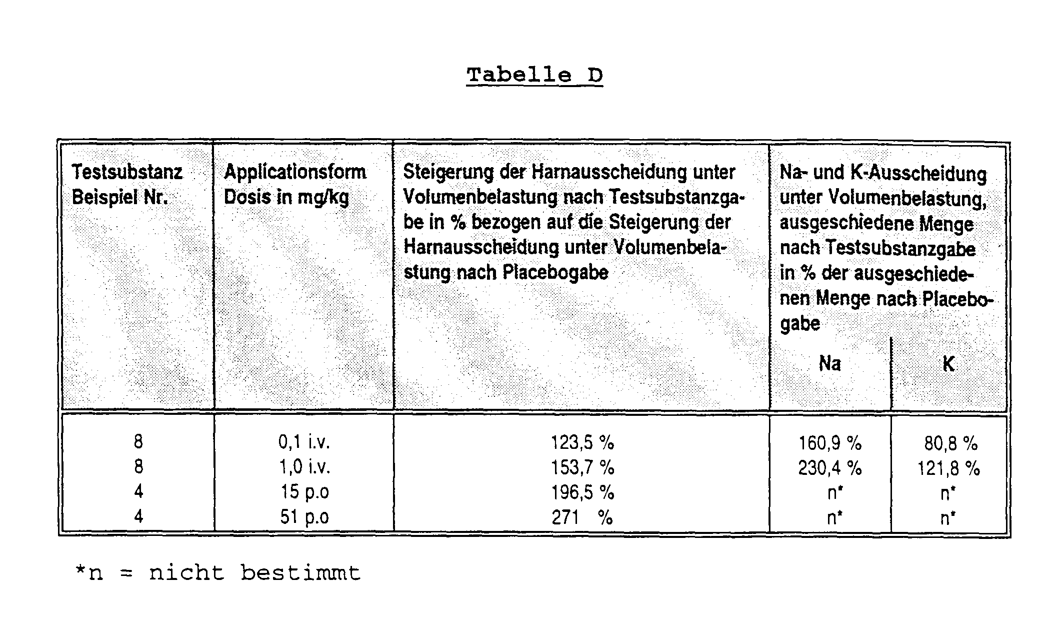

Die vorstehenden Testergebnisse zeigen, daß die Verbindungen der Formel I eine hohe Affinität zu NEP besitzen und durch Hemmung dieses ANP-abbauenden Enzyms zu einer Erhöhung des ANP-Spiegels im Blut beitragen und dadurch dosisabhängig die durch ANP ausgelösten diuretisch/natriuretischen Effekte erhöhen ohne einen wesentlichen Kaliumverlust zu verursachen.

Aufgrund ihrer vorstehend beschriebenen Wirkung sind die Verbindungen der Formel I geeignet als Arzneimittel für größere Säugetiere, insbesondere Menschen, zur Behandlung von Herzinsuffizienz und zur Förderung der Diurese/Natriurese, insbesondere bei an Herzinsuffizienz leidenden Patienten. Hierbei werden Dicarbonsäuren der Formel I und deren Salze zweckmäßig in parenteral, insbesondere i.v., applizierbaren Arzneiformen und Mono- oder Diester der Formel I zweckmäßig in oral applizierbaren Arzneiformen eingesetzt. Die zu verwendenden Dosen können individuell verschieden sein und variieren naturgemäß je nach Art des zu behandelnden Zustandes, der verwendeten Substanz und der Applikationsform. Zum Beispiel werden parenterale Formulierungen im allgemeinen weniger Wirkstoff enthalten als orale Präparate. Im allgemeinen eignen sich jedoch für Applikationen an größeren Säugetieren, insbesondere Menschen, Arzneiformen mit einem Wirkstoffgehalt von 1 bis 200 mg pro Einzeldosis.

Als Heilmittel können die Verbindungen der Formel I mit üblichen pharmazeutischen Hilfsstoffen in galenischen Zubereitungen, wie z. B. Tabletten, Kapseln, Suppositorien oder Lösungen, enthalten sein. Diese galenischen Zubereitungen können nach an sich bekannten Methoden hergestellt werden unter Verwendung üblicher fester oder flüssiger Trägerstoffe wie z. B. Milchzucker, Stärke oder Talkum oder flüssigen Paraffinen und/oder unter Verwendung von üblichen pharmazeutischen Hilfsstoffen, beispielsweise Tablettensprengmitteln, Lösungsvermittlern oder Konservierungsmitteln.

Die nachfolgenden Beispiele sollen die Erfindung näher erläutern, jedoch deren Umfang in keiner Weise beschränken.

Die Strukturen der neuen Verbindungen wurden durch spektroskopische Untersuchungen, insbesondere durch Analyse der NMR-, Massen-, IR-/oder UV-Spektren und gegebenenfalls Bestimmung der optischen Drehwerte gesichert.

### Beispiel 1:

### 3-{1-[2'-(Ethoxycarbonyl)-4'-phenyl-butyl]-cyclopentan-1-carbonylamino}-2,3,4,5-tetrahydro-2-oxo-1H-1-benzazepin-1-essigsäure-tert.-butylester.

A) Zu einer Lösung von 160,1 g Malonsäurediethylester in einem Liter Dimethylformamid wurden portionsweise 123,4 g Kalium-tert.-butylat bei einer Temperatur von 15 °C zugegeben. Das Reaktionsgemisch wurde 30 Minuten gerührt, dann wurde bei Raumtemperatur eine Lösung von 207,7 g Phenethylbromid in 200 ml Dimethylformamid zugetropft. Anschließend wurde das Reaktionsgemisch eine Stunde auf 60 °C gewärmt und wieder abkühlen gelassen. Das Dimethylformamid wurde unter vermindertem Druck abgedampft, der verbleibende Rückstand wurde in einem Gemisch aus Methyl-tert.butylether und Wasser aufgenommen. Die organische Phase wurde abgetrennt, mit Wasser gewaschen über Natriumsulfat getrocknet und eingedampft. Das als öliger Rückstand verbleibende Rohprodukt wurde durch Destillation unter vermindertem Druck gereinigt. Es wurden 202,5 g 2-Ethoxycarbonyl-4-phenyl-butansäure-ethylester erhalten, KP_{1,5} = 148 - 153 °C.
B) Zu einer Lösung von 23,6 g des vorstehend erhaltenen Diesterproduktes in 285 ml Ethanol wurde eine Lösung von 6,17 g Kaliumhydroxid in 76 ml Wasser unter Eiskühlung zugegeben. Das Reaktionsgemisch wurde mehrere Stunden bei Raumtemperatur gerührt. Anschließend wurde das Ethanol unter vermindertem Druck abgedampft und der Rückstand in einem Gemisch aus Methyl-tert.-butylether und Wasser aufgenommen. Die organische Phase wurde abgetrennt und verworfen und die wäßrige Phase wurde unter Eiskühlung mit verdünnter wäßriger Salzsäure angesäuert und anschließend mehrmals mit Methyl-tert-butylether extrahiert. Die vereinigten Methyl-tert.-butylether-Phasen wurden mit Wasser gewaschen, über Natriumsulfat getrocknet und unter vermindertem Druck eingedampft. Es wurden 20,2 g rohes öliges 2-Carboxy-4-phenylbutansäure-ethylester erhalten, welches ohne weitere Reinigung weiterverarbeitet wurde.
C) Zu 20,2 g des vorstehend erhaltenen Produktes wurden unter Eiskühlung nacheinander 11 ml 35 %-ige wäßrige Formaldehyd-Lösung und 9,23 ml Piperidin zugegeben. Das Reaktionsgemisch wurde mehrere Stunden bei Raumtemperatur gerührt, dann mit Methyl-tert.-butylether verdünnt, mit wäßriger Kaliumhydrogensulfat-Lösung und mit Wasser gewaschen, über Natriumsulfat getrocknet und eingedampft. Der Rückstand wurde unter vermindertem Druck getrocknet. Es wurden 14,8 g α-(2-Phenylethyl)-acrylsäureethylester erhalten.
D) Unter Stickstoffatmosphäre wurden 25,2 ml Diisopropylamin in 150 ml absolutem Tetrahydrofuran gelöst und auf -35 °C abgekühlt. Zu der Lösung wurden 100 ml einer 1,6-normalen Butyllithium-Lösung in n-Hexan zugetropft. Dann wurde das Reaktionsgemisch 30 Minuten bei 0 °C gerührt und anschließend wurde eine Lösung von 8,1 ml Cyclopentancarbonsäure in 20 ml absolutem Tetrahydrofuran zugetropft. Das Reaktionsgemisch wurde 2 Stunden bei 0 °C gerührt. Dann wurde eine Lösung von 16,8 g des unter C) erhaltenen Acrylesters in 20 ml absolutem Tetrahydrofuran zugetropft, das Reaktionsgemisch 2 Stunden bei 0 °C und anschließend mehrere Stunden bei -15 °C stehengelassen. Zur Aufarbeitung wurde das Reaktionsgemisch mit 10 %-iger wäßriger Salzsäure-Lösung angesäuert und mit n-Hexan extrahiert. Die organische Phase wurde siebenmal mit halbgesättigter wäßriger Natriumbicarbonat-Lösung und einmal mit Wasser gewaschen, über Natriumsulfat getrocknet und unter vermindertem Druck eingedampft. Das als Rückstand erhaltene Rohprodukt wurde durch Flash-Chromatographie an Kieselgel unter Verwendung von n-Hexan/ Essigsäureethylester (8:2) gereinigt. Es wurden 19,6 g reines 1-[2'-(Ethoxycarbonyl)-4'-phenyl-butyl]-cyclopentan-1-carbonsäure mit einem Schmelzpunkt von 68 bis 69 °C erhalten.
E) Zu einer Lösung von 100 g α-Tetralon in 820 ml Methanol wurden unter Eiskühlung 108,3 g Brom langsam zugetropft. Danach wurde das Reaktionsgemisch 30 Minuten bei Raumtemperatur gerührt und dann wurden zunächst 122,4 g Hydroxylamin-hydrochlorid und anschließend 110 ml Wasser bei Raumtemperatur zugesetzt. Das Gemisch wurde 3 Tage bei Raumtemperatur gerührt. Dann wurden weitere 493 ml Wasser zugefügt, wobei nach einer Stunde ein weißer Niederschlag ausfiel. Das Reaktionsgemisch wurde weitere 3 Tage gerührt und dann auf 5 °C abgekühlt. Der Niederschlag wurde abgesaugt, mit Wasser gewaschen und bei 40 °C unter vermindertem Druck getrocknet. Es wurden 136,7 g 2-Brom-3,4-Dihydronaphthalin-1(2H)-on-oxim mit einem Schmelzpunkt von 130 bis 132 °C erhalten.
F) 79,5 g des vorstehend erhaltenen Oxims wurden portionsweise zu 452 g auf 80 °C erwärmter Polyphosphorsäure gegeben und das Reaktionsgemisch wurde 18 Stunden bei 80 °C gerührt. Anschließend wurde vorsichtig mit 710 ml Wasser verdünnt und das Gemisch 2 Stunden bei Raumtemperatur gerührt. Der entstandene Niederschlag wurde abgesaugt, mit Wasser, wäßriger Natriumbicarbonat-Lösung, nochmals mit Wasser und abschließend mit Methyl-tert.-butylether gewaschen und über Kaliumhydroxid bei einer Temperatur von 60 °C getrocknet. Es wurden 66,6 g 3-Brom-4,5-dihydro-1H-1-benzazepin-2 (3H)-on mit einem Schmelzpunkt von 168 bis 170 °C erhalten.
G) 80 g des vorstehend erhaltenen Produktes wurden in 140 ml Dimethylformamid suspendiert. Die Suspension wurde mit einer Lösung von 72,6 g Kaliumphthalimid in 205 ml Dimethylformamid versetzt und anschließend 16 Stunden bei 60 °C gerührt. Zur Aufarbeitung wurde auf Raumtemperatur abgekühlt, und es wurden langsam 800 ml Wasser zugetropft und das Gemisch 2 Stunden unter Eiskühlung gerührt. Der entstandene Kristallbrei wurde abgesaugt, und erst mit einem Wasser/Dimethylformamid-Gemisch und dann mit Methyl-tert.-butylether gewaschen und anschließend 2 Tage bei 60 °C unter vermindertem Druck getrocknet. Es wurden 73,3 g 4,5-Dihydro-3-phthalimido-1H-1-benzazepin-2(3H)-an mit einem Schmelzbereich von 185 bis 195 °C erhalten.
H) Zu einer Suspension von 27 g des vorstehend erhaltenen Produktes in 90 ml Dimethylformamid wurde unter Eiskühlung eine Lösung von 12,3 g Kalium-tert.-butylat in 40 ml Dimethylformamid gegeben. Nach 30-minütigem Rühren unter Eiskühlung wurden 20,7 g Bromessigsäure-tert.-butylester über eine Stunde verteilt bei 0 bis 5 °C zugetropft. Es wurde eine Stunde bei 0 °C gerührt. Dann wurde das Reaktionsgemisch auf 40 °C erwärmt, und es wurden 164 ml Wasser innerhalb von 3 Stunden zugetropft und noch eine Stunde bei 30 °C gerührt. Dann wurde die wäßrige Lösung von dem gebildeten Niederschlag abdekantiert und der verbleibende feste Rückstand wurde aus Methyl-tert.-butylether kristallisiert. Die gebildeten Kristalle wurden abgesaugt, mit Wasser und mit Methyl-tert.-butylether gewaschen und unter vermindertem Druck bei 60 °C getrocknet. Es wurden 26,3 g 2,3,4,5-Tetrahydro-2-oxo-3-phthalimido-1H-1-benzazepin-1-essigsäure-tert.-butylester mit einem Schmelzpunkt von 194 - 197 °C erhalten.
I) 7 g des vorstehend erhaltenen Esters wurden innerhalb von 5 Minuten zu 13,8 ml auf 80 °C erwärmtem Ethanolamin gegeben. Nach 5 Minuten hatte sich eine klare Lösung gebildet, welche auf Raumtemperatur abgekühlt und mit 105 ml Toluol verdünnt wurde. Die Lösung wurde mit 140 ml 5 %-iger wäßriger Natriumchlorid-Lösung ausgeschüttelt, die organische Phase wurde abgetrennt, über Natriumsulfat getrocknet und eingedampft. Der verbleibende Rückstand wurde aus Methyl-tert.-butylether kristallisiert. Es wurden 4,0 g 3-Amino-2,3,4,5-tetrahydro-2-oxo-1H-1-benzazepin-1-essigsäure-tert.-butylester mit einem Schmelzpunkt von 117 bis 118 °C erhalten.
J) 2,9 g des vorstehend erhaltenen Amins und 3,2 g der vorstehend unter D) erhaltenen Säure wurden in 100 ml Dichlormethan gelöst. Zu dem Reaktionsgemisch wurden 2,2 ml N-Methylmorpholin, 1,27 g Hydroxybenzotriazol und 3,81 g N-Ethyl-N-(3-dimethylaminopropyl)-carbodiimid-hydrochlorid unter Eiskühlung zugefügt. Dann wurde das Reaktionsgemisch 1 Stunde bei Raumtemperatur gerührt. Zur Aufarbeitung wurde das Reaktionsgemisch mit Dichlormethan verdünnt und nacheinander mit Wasser, wäßriger Kaliumhydrogensulfat-Lösung, Wasser, wäßriger Natriumbicarbonatlösung und erneut mit Wasser gewaschen. Die organische Phase wurde sodann über Natriumsulfat getrocknet und das Lösungsmittel wurde unter vermindertem Druck abgedampft. Das so erhaltene Rohprodukt wurde mittels Säulenchromatographie an Kieselgel unter leicht erhöhtem Druck (= Flash-Chromatographie) unter Verwendung von n-Hexan/Essigsäureethylester gereinigt, wobei der Essigsäureester-Anteil des Elutionsmittels während der Elution von anfangs 1:9 auf 3:7 erhöht wurde. Es wurden 5,4 g der reinen Titelverbindung als öliges Produkt erhalten.

- IR-Spektrum (als Film) :: 3400 cm⁻¹, 1725 cm⁻¹, 1660 cm⁻¹

### Beispiel 2:

### 3-{1-[2'-(Ethoxycarbonyl)-4'-phenyl-butyl]-cyclopentan-1-carbonylamino}-2,3,4,5-tetrahydro-2-oxo-1H-1-benzazepin-1-essigsäure.

5 g 3-{1-[2'-(Ethoxycarbonyl)-4'-phenyl-butyl]-cyclopentan-1-carbonylamino}-2,3,4,5-tetrahydro-2-oxo-1H-1-benzazepin-1-essigsäure-tert.-butylester (Herstellung siehe Beispiel 1) wurden in 16 ml Trifluoressigsäure gelöst. Die Lösung wurde 3 Stunden bei Raumtemperatur gerührt. Zur Aufarbeitung wurde die Trifluoressigsäure unter vermindertem Druck abgedampft. Der verbleibende Rückstand wurde in Dichlormethan gelöst und die Lösung wurde mit Wasser neutral gewaschen. Anschließend wurde die organische Phase über Natriumsulfat getrocknet und unter vermindertem Druck eingedampft. Der verbleibende Rückstand wurde mehrfach mit n-Hexan verrührt und jeweils wieder zur Trockene eingedampft. Es wurden 3,4 g der Titelverbindung als fester Schaum mit einem Schmelzbereich von 81 bis 104 °C erhalten.

### Beispiel 3:

### (3S,2'R)-3-{1-[2'-(Ethoxycarbonyl)-4'-phenyl-butyl]-cyclopentan-1-carbonylamino}-2,3,4,5-tetrahydro-2-oxo-lH-1-benzazepin-1-essigsäure-tert.-butylester.

A) 30,5 g 1-[2'-(Ethoxycarbonyl)-4'-phenyl-butyl]-cyclopentan-1-carbonsäure (Herstellung siehe Beispiel 1D)) und 11,6 g L-(-)-α-Methylbenzylamin wurden in Ethanol unter Erwärmung gelöst. Das Reaktionsgemisch wurde 12 Stunden im Kühlschrank gekühlt, dann wurde der ausgefallene Kristallbrei abgesaugt, getrocknet und mehrmals (bis zur Drehwertkonstanz) aus Ethanol umkristallisiert und anschließend unter vermindertem Druck getrocknet. Es wurden 17,7 g eines α-Methylbenzylammoniumsalzes der vorstehenden Säure mit einem Schmelzpunkt von 118 bis 121 °C erhalten, optischer Drehwert [α]²⁰_{D} = +5,6° (c = 0,5 in Methanol).
   Zur Freisetzung der Säure wurde dieses Salz in einem Wasser/Dichlormethan-Gemisch aufgenommen und das Gemisch wurde mit wäßriger Kaliumhydrogensulfat-Lösung sauergestellt. Die organische Phase wurde abgetrennt und die wäßrige Phase noch dreimal mit Dichlormethan extrahiert. Die vereinigten organischen Extrakte wurden mit Wasser gewaschen, über Natriumsulfat getrocknet und unter vermindertem Druck eingedampft. Der verbleibende Rückstand wurde getrocknet. Es wurden 11,2 g reine (2'R)-1-[2'-(Ethoxycarbonyl-4'-phenylbutyl]-cyclopentan-1-carbonsäure erhalten, optischer Drehwert [α]²⁰_{D} = +7,4° (c = 0,651 in Methanol).
B) Zu einer auf 65 °C erhitzten Lösung von 24,5 g des racemischen 3-Amino-2,3,4,5-tetrahydro-2-oxo-1H-1-benzazepin-1-essigsäure-tert.-butylester (Herstellung siehe Beispiel 1I) wurde eine Lösung von 12,65 g L-(+)-Weinsäure in 54 ml auf 65 °C erhitztem Ethanol gegeben. Das Reaktionsgemisch wurde eine Stunde bei Raumtemperatur gerührt. Dann wurde eine Lösung von 1,72 ml Benzaldehyd in 1,3 ml Ethanol hinzugetropft. Die erhaltene Suspension wurde 14 Stunden bei 80 °C am Rückfluß gekocht und dann auf Raumtemperatur abgekühlt. Der entstandene kristalline Niederschlag wurde abgesaugt, in 80 ml Ethanol aufgenommen und erneut 8 Stunden am Rückfluß gekocht. Dann wurde auf Raumtemperatur abgekühlt und die Kristalle wurden abgesaugt und unter vermindertem Druck bei 50 °C getrocknet. Es wurden 23,6 g weinsaures Salz mit einem Schmelzpunkt von 195 bis 196 °C und einem optischen Drehwert [α]²⁰_{D} von -152° (c=0,5 in Methanol) erhalten.
   Zur Freisetzung der Base wurden 23,6 g des weinsauren Salzes in einem Gemisch aus 250 ml Wasser und 108 ml Dichlormethan unter Rühren auf 0 °C abgekühlt und durch Zusatz von wäßriger Ammoniak-Lösung auf pH 9,6 eingestellt. Die organische Phase wurde abgetrennt, die wäßrige Phase nochmals mit 30 ml Dichlormethan extrahiert und die organischen Phasen vereinigt, über Natriumsulfat getrocknet und unter vermindertem Druck eingeengt. Der verbleibende Rückstand wurde aus Methyl-tert.-butylether kristallisiert und unter vermindertem Druck getrocknet. Es wurden 12,2 g (3S)-3-Amino-2,3,4,5-tetrahydro-2-oxo-1H-1-benzazepin-1-essigsäure-tert.-butylester mit einem Schmelzpunkt von 113 bis 115 °C und einem optischen Drehwert [α]²⁰_{D} von -276,2° (c=0,5 in Methanol) erhalten.
C) 5,4 g der vorstehend unter A) hergestellten Säure wurden in 60 ml trockenem Dichlormethan gelöst. Die Lösung wurde mit 2,33 ml Triethylamin versetzt und auf -20 °C abgekühlt. Dann wurde langsam eine Lösung von 1,31 ml Methansulfonsäurechlorid in 5 ml trockenem Dichlormethan zugetropft. Nach 15-minütigem Rühren wurde eine Lösung von 4,8 g des vorstehend unter B) erhaltenen Amins und 2,33 ml Triethylamin in 60 ml Dichlormethan zugetropft. Anschließend wurde das Reaktionsgemisch 1 Stunde bei Raumtemperatur gerührt. Zur Aufarbeitung wurde das Reaktionsgemisch in Wasser gegeben, die organische Phase abgetrennt und mit wäßriger Kaliumhydrogensulfat-Lösung und anschließend mit Wasser gewaschen, über Natriumsulfat getrocknet, filtriert und unter vermindertem Druck eingeengt. Das verbleibende Rohprodukt wurde durch Flash-Chromatographie an 500 g Kieselgel unter Verwendung von n-Hexan/Essigsäureethylester (7:3) gereinigt. Nach Trocknen unter vermindertem Druck wurden 9,5 g reine Titelverbindung als Öl erhalten, optischer Drehwert [α]²⁰_{D} = -115,2° (c = 0,463 in Methanol).

### Beispiel 4:

### (3S,2'R)-3-{1-[2'-(Ethoxycarbonyl)-4'-phenyl-butyl]-cyclopentan-1-carbonylamino}-2,3,4,5-tetrahydro-2-oxo-1H-1-benzazepin-1-essigsäure.

9,4 g (3S,2'R)-3-{1-[2'-(Ethoxycarbonyl)-4'-phenyl-butyl]-cyclopentan-1-carbonylamino}-2,3,4,5-tetrahydro-2-oxo-1H-1-benzazepin-1-essigsäure-tert.-butylester (Herstellung siehe Beispiel 3) wurden unter Eiskühlung in 15 ml Dichlormethan gelöst. Die Lösung wurde mit 31 ml Trifluoressigsäure versetzt und das Reaktionsgemisch wurde ca. 12 Stunden bei 4 °C im Kühlschrank gehalten. Zur Aufarbeitung wurden das Dichlormethan und die Trifluoressigsäure unter vermindertem Druck abgedampft. Das erhaltene Rohprodukt wurde in Essigsäureethylester aufgenommen, mit Wasser, verdünnter wäßriger Natriumbicarbonatlösung und erneut mit Wasser gewaschen. Die organische Phase wurde abgetrennt, über Natriumsulfat getrocknet und unter vermindertem Druck eingedampft. Der verbleibende Rückstand wurde durch Flash-Chromatographie an Kieselgel gereinigt, wobei als Elutionsmittel zunächst Dichlormethan und dann Dichlormethan/Methanol (95:5) verwendet wurde. Das erhaltene Produkt wurde 2 Tage bei 80 °C unter vermindertem Druck getrocknet. Es wurden 7,3 g der reinen Titelverbindung als fester Schaum mit einem Schmelzpunkt von 71 bis 74 °C erhalten, optischer Drehwert [α]²⁰_{D} = -131,0° (c = 0,5 in Methanol).

### Beispiel 5:

### 3-{1-[2'-(tert.-Butoxycarbonyl)-4'-phenyl-butyl]-cyclopentan-1-carbonylamino}-2,3,4,5-tetrahydro-2-oxo-1H-1-benzazepin-1-essigsäure-tert.butylester.

A) 118 g Dimethylphosphonoessigsäure-tert.-butylester wurden unter Stickstoffatmosphäre in 875 ml trockenem Dimethylformamid gelöst. Zu der Lösung wurden 58,9 g Kalium-tert.-butylat unter Eiskühlung gegeben. Anschließend wurde das Reaktionsgemisch für kurze Zeit auf 60 °C erhitzt und dann auf Raumtemperatur abkühlen gelassen. Zu dem Reaktionsgemisch wurde eine Lösung von 104,9 g Phenethylbromid in 110 ml Dimethylformamid zugetropft. Dann wurde das Reaktionsgemisch für 2 Stunden auf 60 °C erhitzt. Zur Aufarbeitung wurde das Dimethylformamid unter vermindertem Druck weitgehend abgedampft und der verbliebene Rückstand wurde in Methyl-tert.-butylether gelöst. Die Lösung wurde mit wäßriger Kaliumhydrogensulfat-Lösung angesäuert. Dann wurde die organische Phase abgetrennt, mit Wasser gewaschen, über Natriumsulfat getrocknet und unter vermindertem Druck eingedampft. Das erhaltene Rohprodukt wurde durch Flash-Chromatographie an 3 kg Kieselgel unter Verwendung von Dichlormethan/Methyl-tert.-butylether (4:1) als Elutionsmittel gereinigt. Es wurden 105,1 g reines 2-(Dimethylphosphono)-4-phenyl-n-buttersäure-tert.-butylester als öliges Produkt erhalten.
B) 105,1 g des vorstehend erhaltenen Produktes wurden unter Stickstoffatmosphäre in 705 ml trockenem Tetrahydrofuran gelöst. Zu der Lösung wurden 28,4 g Paraformaldehyd gegeben. Dann wurde langsam eine Lösung von 32,5 g Kalium-tert.-butylat in 100 ml Tetrahydrofuran zugetropft. Anschließend wurde das Reaktionsgemisch 1 Stunde gerührt. Zur Aufarbeitung wurde das Reaktionsgemisch mit kalter wäßriger Kaliumhydrogensulfat-Lösung angesäuert und mit Methyl-tert.-butylether verdünnt. Dann wurde die organische Phase abgetrennt, mit Wasser gewaschen, über Natriumsulfat getrocknet und unter vermindertem Druck eingeengt. Das erhaltene Rohprodukt wurde durch Flash-Chromatographie an 700 g Kieselgel unter Verwendung von n-Hexan/Essigsäureethylester (9:1) gereinigt. Es wurden 47,0 g α-(Phenethyl)-acrylsäure-tert.-butylester als farbloses Öl erhalten.
C) Zu einer auf -50 °C abgekühlten Lösung von 50,2 ml Diisopropylamin in 450 ml absolutem Tetrahydrofuran wurden 200 ml einer 1,6-molaren Lösung von Butyllithium in n-Hexan getropft und das Reaktionsgemisch wurde weitere 30 Minuten bei 0 °C gehalten. Anschließend wurde bei dieser Temperatur eine Lösung von 16,2 ml Cyclopentancarbonsäure in 40 ml absolutem Tetrahydrofuran zugetropft. Das Reaktionsgemisch wurde weitere 2 Stunden bei 0 °C gerührt. Dann wurde zu dem Gemisch langsam eine Lösung von 38 g des vorstehend unter B) erhaltenen Produktes in 50 ml absolutem Tetrahydrofuran zugegeben. Das Reaktionsgemisch wurde weitere 2 Stunden bei 0 °C gerührt und dann noch mehrere Stunden bei -15 °C stehengelassen. Zur Aufarbeitung wurde das Reaktionsgemisch unter Eiskühlung mit gesättigter wäßriger Kaliumhydrogensulfat-Lösung angesäuert und dreimal mit n-Hexan extrahiert. Die vereinigten organischen Phasen wurden siebenmal mit halbgesättigter wäßriger Natriumbicarbonat-Lösung und anschließend mit Wasser gewaschen, dann über Natriumsulfat getrocknet und unter vermindertem Druck eingedampft. Das erhaltene ölige Rohprodukt wurde aus eiskaltem n-Hexan kristallisiert. Es wurden 41,9 g reine kristalline 1-[2'-(tert.-Butoxycarbonyl)-4'-phenyl-butyl]-cyclopentan-1-carbonsäure mit einem Schmelzpunkt von 75 bis 77 °C erhalten.
D) 3,3 g des vorstehend erhaltenen Produktes, 2,7 g 3-Amino-2,3,4,5-tetrahydro-2-oxo-1H-1-benzazepin-1-essigsäure-tert.-butylester (Herstellung siehe Beispiel 1I)), 1,53 ml N-Methylmorpholin und 1,18 g Hydroxybenzotriazol wurden unter Stickstoffatmosphäre in 93 ml absolutem Dichlormethan gelöst. Zu dieser Lösung wurden unter Eiskühlung 3,52 g N-Ethyl-N'-(3-dimethylaminopropyl)-carbodiimid-hydrochlorid zugefügt. Dann wurde das Reaktionsgemisch 2 Stunden unter Eiskühlung gerührt. Zur Aufarbeitung wurde das Reaktionsgemisch nacheinander mit Wasser, wäßriger Kaliumhydrogensulfat-Lösung, Wasser, wäßriger Natriumbicarbonat-Lösung und wiederum Wasser gewaschen. Die organische Phase wurde über Natriumsulfat getrocknet und unter vermindertem Druck eingeengt. Das verbleibende Rohprodukt wurde durch Flash-Chromatographie an 200 g Kieselgel unter Verwendung von n-Hexan/Essigsäureethylester (7:3) als Elutionsmittel gereinigt und aus Methyl-tert.-butylether kristallisiert. Es wurden 4,2 g der reinen Titelverbindung mit einem Schmelzpunkt von 110 bis 114 °C erhalten.

### Beispiel 6:

### 3-[1-(2'-Carboxy-4'-phenyl-butyl)-cyclopentan-1-carbonylamino]-2,3,4,5-tetrahydro-2-oxo-1H-1-benzazepin-1-essigsäure.

4,1 g 3-{1-[2'-(tert.-Butoxycarbonyl)-4'-phenyl-butyl]-cyclopentan-1-carbonylamino}-2,3,4,5-tetrahydro-2-oxo-1H-1-benzazepin-1-essigsäure-tert.-butylester (Herstellung siehe Beispiel 5) wurden in 13 ml Trifluoressigsäure bei einer Temperatur von 4 °C unter Feuchtigkeitsausschluß gelöst. Die erhaltene Lösung wurde weitere 3 Stunden bei dieser Temperatur gerührt. Zur Aufarbeitung wurde das Reaktionsgemisch unter vermindertem Druck eingeengt. Zur völligen Entfernung von Trifluoressigsäure wurde der Rückstand mehrfach mit Dichlormethan versetzt und wieder eingedampft. Der erhaltene Rückstand wurde dann in Dichlormethan gelöst, die Lösung mit Wasser gewaschen, über Natriumsulfat getrocknet und unter vermindertem Druck eingeengt. Das als Rückstand verbleibende Rohprodukt wurde aus Dichlormethan kristallisiert. Es wurden 2,7 g der reinen Titelverbindung mit einem Schmelzpunkt von 178 bis 183 °C erhalten.

### Beispiel 7:

### (3S,2'R)-3-{1-[2'-(tert.-Butoxycarbonyl)-4'-phenyl-butyl]-cyclopentan-1-carbonylamino}-2,3,4,5-tetrahydro-2-oxo-1H-1-benzazepin-1-essigsäure-tert.-butylester.

A) 68 g 1-[2'-(tert.-Butoxycarbonyl)-4'-phenyl-butyl]-cyclopentan-1-carbonsäure (Herstellung siehe 5C)) und 23,5 ml L-(-)-α-Methylbenzylamin wurden in 200 ml Ethanol unter Erwärmen gelöst. Das Reaktionsgemisch wurde wie in Beispiel 3A) beschrieben aufgearbeitet. Es wurden 32,2 g eines α-Methylbenzylammoniumsalzes der vorstehenden Säure mit einem Schmelzpunkt von 118 bis 119 °C erhalten, optischer Drehwert [α]²⁰_{D} = +9,2° (c = 0,5 in Methanol). Zur Freisetzung der Säure wurde dieses Salz nach dem in Beispiel 3A) beschriebenen Verfahren weiterbehandelt. Es wurden 23 g (2'R)-1-[2'-(tert.-Butoxycarbonyl)-4'-phenyl-butyl]-cyclopentan-1-carbonsäure mit einem Schmelzpunkt von 68 bis 70 °C erhalten, optischer Drehwert [α]²⁰_{D} = +15,4° (c = 0,5 in Methanol).
B) 60,1 g der vorstehend erhaltenen Säure wurden mit 50,3 g (3S)-3-Amino-2,3,4,5-tetrahydro-2-oxo-1H-1-benzazepin-1-essigsäure-tert.-butylester (Herstellung siehe Beispiel 3B)) nach der in Beispiel 3C) beschriebenen Methode umgesetzt und das erhaltene Reaktionsgemisch wurde wie in Beispiel 3C) beschrieben aufgearbeitet. Es wurden 94,3 g der Titelverbindung als Schaum erhalten, optischer Drehwert [α]²⁰_{D} = -110,2° (c = 0,5 in Methanol).
   - IR-Spektrum (als KBr-Preßling) :: 3420 cm⁻¹, 1743 cm⁻¹,
   1725 cm⁻¹, 1670 cm⁻¹

### Beispiel 8:

### (3S,2'R)-3-[1-(2'-Carboxy-4'-phenyl-butyl)-cyclopentan-1-carbonylamino]-2,3,4,5-tetrahydro-2-oxo-1H-1-benzazepin-1-essigsäure.

93 g (3S,2'R)-3-{1-[2'-(tert.-Butoxycarbonyl)-4'-phenyl-butyl]-cyclopentan-1-carbonylamino}-2,3,4,5-tetrahydro-2-oxo-1H-1-benzazepin-1-essigsäure-tert.-butylester (Herstellung siehe Beispiel 7) wurden nach der in Beispiel 6 beschriebenen Methode mit Trifluoressigsäure hydrolysiert und das Reaktionsgemisch wurde wie in Beispiel 6 beschrieben aufgearbeitet. Es wurden 63,5 g der Titelverbindung als fester Schaum mit einem Schmelzbereich von 97 bis 122 °C erhalten, optischer Drehwert [α]²⁰_{D} = -136,2° (c = 0,5 in Methanol).

### Beispiel 9:

### (3S,2'S)-3-{1-[2'-(tert.-Butoxycarbonyl)-3'-(2-methoxyethoxy)-propyl]-cyclopentan-1-carbonylamino}-3,4-dihydro-4-oxo-1,5-benzoxazepin-5(2H)-essigsäure-benzylester.

A) Zu einer Lösung von 100 g 3-Brompropionsäure in 200 ml Diethylether wurden 7 ml Schwefelsäure gegeben und das Reaktionsgemisch wurde auf -20 °C abgekühlt. Dann wurden 123 ml verflüssigtes Isobuten zugegeben. Das Reaktionsgemisch wurde mehrere Stunden bei Raumtemperatur in einem Druckgefäß gerührt. Anschließend wurde das Reaktionsgemisch zur Aufarbeitung in verdünnte eiskalte wäßrige Natronlauge gegeben. Die Etherphase wurde abgetrennt und die wäßrige Phase nochmals mit Ether extrahiert. Die vereinigten organischen Phasen wurden mit wäßriger Natriumchlorid-Lösung gewaschen, über Natriumsulfat getrocknet und unter vermindertem Druck eingedampft. Das erhaltene Rohprodukt wurde unter vermindertem Druck destilliert. Es wurden 100 g 3-Brompropionsäure-tert.-butylester, KP₂₀ = 75 bis 77 °C, erhalten.
B) 50,4 ml Diisopropylamin wurden in 300 ml absolutem Tetrahydrofuran unter Stickstoffatmosphäre gelöst und die Lösung wurde auf -70 °C abgekühlt. Zu der Lösung wurden bei dieser Temperatur 200 ml einer 1,6-molaren Lösung von Butyllithium in n-Hexan langsam zugetropft. Das Reaktionsgemisch wurde sich auf 0 °C erwärmen gelassen, 30 Minuten bei dieser Temperatur gerührt und wieder auf -20 °C abgekühlt. Bei dieser Temperatur wurde eine Lösung von 16,2 ml Cyclopentancarbonsäure in 30 ml absolutem Tetrahydrofuran zugetropft. Dann wurde das Reaktionsgemisch 2 Stunden bei Raumtemperatur gerührt. Anschließend wurde das Gemisch auf -10 °C abgekühlt und langsam zu einer auf -10 °C abgekühlten Lösung von 35 g 3-Brompropionsäure-tert.-butylester in 100 ml Tetrahydrofuran getropft. Das Reaktionsgemisch wurde mehrere Stunden bei Raumtemperatur gerührt. Zur Aufarbeitung wurde mit verdünnter wäßriger Salzsäurelösung angesäuert und mit 375 ml Diethylether verdünnt. Die organische Phase wurde abgetrennt und die wäßrige Phase wurde noch dreimal mit je 100 ml Diethylether extrahiert. Die vereinigten organischen Extrakte wurden mit wäßriger Natriumchloridlösung gewaschen, über Natriumsulfat getrocknet und unter vermindertem Druck eingedampft. Der verbleibende Rückstand wurde in 300 ml Diethylether gelöst. Die Lösung wurde sechsmal mit wäßriger Natriumbicarbonatlösung und anschließend viermal mit 10 %-iger wäßriger Natriumcarbonatlösung geschüttelt. Die vereinigten Natriumcarbonatlösungen wurden unter Eiskühlung angesäuert und dreimal mit je 150 ml Ether extrahiert. Diese Etherextrakte wurden mit der etherischen Lösung vereinigt und die erhaltene Lösung wurde mit wäßriger Natriumchloridlösung gewaschen, über Natriumsulfat getrocknet und unter vermindertem Druck eingeengt. Das erhaltene Rohprodukt wurde aus eiskaltem n-Hexan kristallisiert. Es wurden 7,7 g reines 1-[2'-(tert.-Butoxycarbonyl)-ethyl]-1-cyclopentancarbonsäure mit einem Schmelzpunkt von 78 bis 81 °C erhalten.
C) 30 ml Diisopropylamin wurden in 100 ml absolutem Tetrahydrofuran unter Stickstoffatmosphäre gelöst und die Lösung wurde auf -70 °C abgekühlt. Zu der Lösung wurden 132 ml einer 1,6-molaren Lösung von Butyllithium in n-Hexan getropft und das Reaktionsgemisch wurde weitere 30 Minuten bei 0 °C gerührt und dann erneut auf -70 °C abgekühlt. Anschließend wurden zu dem Reaktionsgemisch nacheinander eine Lösung von 24,2 g des vorstehend unter B) hergestellten Produktes in 100 ml absolutem Tetrahydrofuran und dann eine Lösung von 14,2 ml Methoxyethoxymethylchlorid in 20 ml absolutem Tetrahydrofuran zugetropft. Das Reaktionsgemisch wurde noch 16 Stunden bei Raumtemperatur gerührt. Zur Aufarbeitung wurde das Reaktionsgemisch in ein Eis/Wasser-Gemisch gegeben, mit wäßriger Kaliumhydrogensulfat-Lösung angesäuert und dreimal mit je 300 ml Essigsäureethylester extrahiert. Die organischen Phasen wurden vereinigt, mit wäßriger Natriumchloridlösung gewaschen, über Natriumsulfat getrocknet und unter vermindertem Druck eingeengt. Das verbleibende Rohprodukt wurde durch Flash-Chromatographie an 500 g Kieselgel unter Verwendung von Dichlormethan/Ether (8:2) als Elutionsmittel gereinigt. Es wurden 26,5 g reines 1-[2'-(tert.-Butoxycarbonyl)-3'-(2-methoxyethoxy)-propyl]-cyclopentan-1-carbonsäure als Öl erhalten.
D) 36,7 g der vorstehend erhaltenen racemischen Säure wurden in 184 ml n-Hexan gelöst und die Lösung wurde mit 18,4 g (+)-Pseudoephedrin versetzt. Der ausgefallene Niederschlag wurde durch kurzzeitiges Kochen am Rückfluß wieder in Lösung gebracht. Dann wurde die Lösung abgekühlt und mehrere Stunden im Kühlschrank stehengelassen. Der gebildete kristalline Niederschlag wurde abgesaugt, mit eiskaltem n-Hexan gewaschen und noch viermal aus n-Hexan umkristallisiert. Es wurden 16,2 g eines Pseudoephedrin-Salzes der vorstehenden Säure mit einem Schmelzpunkt von 89 bis 91 °C erhalten, optischer Drehwert [α]²⁰_{D} = +36,5° (c = 1 in Methanol).
   Zur Freisetzung der Säure wurden 16 g dieses Salzes in n-Hexan suspendiert, und das Reaktionsgemisch wurde mit eiskalter wäßriger Kaliumhydrogensulfat-Lösung angesäuert. Die organische Phase wurde abgetrennt, und die wäßrige Phase wurde noch zweimal mit n-Hexan extrahiert. Die vereinigten organischen Phasen wurden mit wäßriger Natriumchlorid-Lösung gewaschen, über Natriumsulfat getrocknet und unter vermindertem Druck eingedampft. Der Rückstand wurde unter vermindertem Druck bei 50 °C getrocknet. Es wurden 9,9 g (2'S)-1-[2'-(tert.-Butoxycarbonyl)-3'-(2-methoxy-ethoxy)-propyl]-cyclopentan-1-carbonsäure als Öl erhalten, optischer Drehwert [α]²⁰_{D} = +2,9° (c = 1 in Methanol).
E) 17,2 g Natriumhydrid (80 %-ig) wurden in 400 ml trockenem Dimethylformamid unter Stickstoffatmosphäre und Feuchtigkeitsausschluß gelöst. Zu dieser Lösung wurde bei 0 °C eine Lösung von 50 g L-BOC-Serin (= N-(tert.-Butoxycarbonyl)serin) in 50 ml trockenem Dimethylformamid langsam zugetropft. Das Reaktionsgemisch wurde sich langsam auf 15 °C erwärmen gelassen, dann wurde eine Lösung von 37,4 g o-Nitrophenol in 50 ml Dimethylformamid zugetropft, und das Reaktionsgemisch wurde noch mehrere Stunden bei Raumtemperatur gerührt. Zur Aufarbeitung wurde das Reaktionsgemisch auf eiskalte wäßrige Kaliumhydrogensulfat-Lösung gegossen. Dann wurde mehrfach mit Essigsäureethylester extrahiert und die vereinten organischen Phasen wurden mit wäßriger Natriumbicarbonat-Lösung versetzt. Die wäßrige Phase wurde abgetrennt, mit Ether gewaschen und anschließend mit Kaliumhydrogensulfat-Lösung angesäuert und mit Essigsäureethylester extrahiert. Der Essigsäureethylesterextrakt wurde mit Wasser gewaschen, über Natriumsulfat getrocknet und unter vermindertem Druck eingedampft. Es wurden 54,2 g rohe (2S)-3-(2-Nitrophenoxy)-2-(tert.-butoxycarbonylamino)-propionsäure erhalten, welche ohne weitere Reinigung weiterverarbeitet wurde.
F) 54,2 g der vorstehend erhaltenen Säure wurden in 600 ml Methanol gelöst. Die Lösung wurde mit 1,8 g Palladiumkatalysator (5 % Pd/Kohle) versetzt. Dann wurde 1 Stunde lang bei einem Wasserstoffdruck von 5 bar hydriert. Anschließend wurde der Katalysator abfiltriert und die filtrierte Lösung unter vermindertem Druck eingeengt. Das erhaltene Rohprodukt wurde aus einem Methyl-tert.-butylether/n-Hexan-Gemisch unter Eiskühlung kristallisiert. Es wurden 30,1 g (2S)-3-(2-Aminophenoxy)-2-(tert.-butoxycarbonylamino)-propionsäure mit einem Schmelzpunkt von 87 bis 91 °C erhalten, optischer Drehwert [α]²⁰_{D} = +55,9° (c = 1 in Methanol).
G) 13,3 g der vorstehend erhaltenen Säure wurden in 71 ml trockenem Dimethylformamid unter Feuchtigkeitsausschluß gelöst. Zu der Lösung wurde unter Eiskühlung eine Lösung von 7,8 ml Diethylphosphonocyanid in 6 ml Dimethylformamid gegeben. Nach 10 Minuten wurden 5,7 ml Triethylamin zugetropft und das Reaktionsgemisch wurde 1 Stunde bei Raumtemperatur gerührt. Dann wurde das Reaktionsgemisch zur Aufarbeitung auf Eiswasser gegossen und mit Methyl-tert.-butylether mehrfach extrahiert. Die vereinigten organischen Phasen wurden getrocknet und unter vermindertem Druck eingedampft. Das als Rückstand verbleibende Rohprodukt wurde aus Ethanol kristallisiert. Es wurden 1,3 g (3S)-3-(tert.-Butoxycarbonylamino)-2,3-dihydro-1,5-benzoxazepin-4 (5H)-on erhalten, optischer Drehwert [α]²⁰_{D} = -194° (c = 1 in Methanol).
H) 16 g des vorstehend erhaltenen Produktes wurden in 313 ml Tetrahydrofuran unter Feuchtigkeitsausschluß gelöst. Zu der Lösung wurden nacheinander eine Lösung von 7,1 g Kalium-tert.-butylat in 30 ml Tetrahydrofuran und eine Lösung von 10,9 ml Bromessigsäurebenzylester in 10 ml Tetrahydrofuran zugetropft. Das Reaktionsgemisch wurde 1 Stunde bei Raumtemperatur gerührt. Anschließend wurde zur Aufarbeitung mit Methyl-tert.-butylether verdünnt, mit Wasser gewaschen, die organische Phase über Natriumsulfat getrocknet und unter vermindertem Druck eingeengt. Das erhaltene Rohprodukt wurde durch Flash-Chromatographie an 500 g Kieselgel unter Verwendung von n-Hexan/Essigsäureethylester (3:2) als Elutionsmittel gereinigt. Es wurden 20,5 g reines (3S)-3-(tert.-Butoxycarbonylamino)-4-oxo-3,4-dihydro-1,5-benzoxazepin-5(2H)-essigsäurebenzylester als Öl erhalten, optischer Drehwert [α]²⁰_{D} = -152° (c = 0,68 in Methanol).
I) 20 g des vorstehend erhaltenen Produktes wurden in 137 ml Dichlormethan gelöst. Die Lösung wurde mit 77 ml Trifluoressigsäure versetzt und 1 Stunde gerührt. Dann wurde unter vermindertem Druck eingeengt, der Rückstand in Dichlormethan gelöst und mit wäßriger Natriumbicarbonatlösung bis zur alkalischen Reaktion versetzt. Die organische Phase wurde abgetrennt, mit Wasser gewaschen, über Natriumsulfat getrocknet und unter vermindertem Druck eingeengt. Es wurden 15,7 g reines (3S)-3-Amino-4-oxo-3,4-dihydro-1,5-benzoxazepin-5(2H)-essigsäurebenzylester erhalten, optischer Drehwert [α]²⁰_{D} = -187,5° (c = 0,536 in Methanol).
J) 15,7 g des vorstehend erhaltenen Produktes wurden in 48 ml trockenem Dichlormethan gelöst und die Lösung wurde bei Raumtemperatur nacheinander mit 1,6 g der vorstehend unter D) hergestellten Säure, 0,79 ml N-Methylmorpholin und 1,83 g N-Ethyl-N'-(3-dimethylaminopropyl)-carbodiimid-hydrochlorid versetzt. Das Reaktionsgemisch wurde noch 1 Stunde bei Raumtemperatur gerührt. Dann wurde zur Aufarbeitung nacheinander mit Wasser, wäßriger Kaliumhydrogensulfat-Lösung, Wasser, wäßriger Natriumbicarbonat-Lösung und erneut Wasser gewaschen, über Natriumsulfat getrocknet und unter vermindertem Druck eingedampft. Das als Rückstand erhaltene Rohprodukt wurde durch Flash-Chromatographie an Kieselgel unter Verwendung von n-Hexan/Essigsäureethylester (7:3) als Elutionsmittel gereinigt. Es wurden 1,8 g der Titelverbindung als Öl erhalten, optischer Drehwert [α]²⁰_{D} = -96,3° (c = 0,326 in Methanol).

### Beispiel 10:

### (3S,2'S)-3-{1-[2'-Carboxy-3'-(2-methoxyethoxy)-propyl]-cyclopentan-1-carbonylamino}-4-oxo-3,4-dihydro-1,5-benzoxazepin-5(2H)-essigsäurebenzylester.

1,6 g (3S,2'S)-3-{1-[2'-(tert.-Butoxycarbonyl)-3'-(2-methoxyethoxy)-propyl]-cyclopentan-1-carbonylamino}-4-oxo-3,4-dihydro-1,5-benzoxazepin-5(2H)-essigsäurebenzylester (Herstellung siehe Beispiel 9) wurden unter Eiskühlung in 5 ml Trifluoressigsäure gelöst. Die Lösung wurde mehrere Stunden bei einer Temperatur von 4 °C stehengelassen. Anschließend wurde zur Aufarbeitung unter vermindertem Druck eingedampft und das als Rückstand verbleibende Rohprodukt wurde durch Flash-Chromatographie an Kieselgel unter Verwendung von Dichlormethan/Methyl-tert.-butylether/Methanol (85:15:5) gereinigt. Nach Trocknung wurden 1,0 g der Titelverbindung als Öl erhalten, optischer Drehwert [α]²⁰_{D} = -117,2° (c = 0,42 in Methanol).

### Beispiel 11:

### (3S,2'S)-3-{1-[2'-Carboxy-3'-(2-methoxyethoxy)-propyl]-cyclopentan-1-carbonylamino}-4-oxo-3,4-dihydro-1,5-benzoxazepin5(2H)-essigsäure.

0,95 g (3S,2'S)-3-{1-[2'-Carboxy-3'-(2-methoxyethoxy)-propyl]-cyclopentan-1-carbonylamino}-4-oxo-3,4-dihydro-1,5-benzoxazepin-5(2H)-essigsäurebenzylester. (Herstellung siehe Beispiel 10) wurden in 50 ml Ethanol gelöst. Zu der Lösung wurden 0,2 g Palladium-Katalysator (Pd/Kohle 5%) gegeben. Dann wurde 2 Stunden bei einem Wasserstoffdruck von 5 bar hydriert. Zur Aufarbeitung wurde von dem Katalysator abfiltriert, die filtrierte Lösung unter vermindertem Druck eingedampft und der verbleibende Rückstand getrocknet. Es wurden 0,7 g der Titelverbindung als schaumiges Produkt erhalten, optischer Drehwert [α]²⁰_{D} = -142,6° (c = 0,5 in Methanol).

### Beispiel 12:

### (3R)-3-{1-[2'-(tert.-Butoxycarbonyl)-4'-(4-fluorphenoxy)-butyl]-cyclopentan-1-carbonylamino}-4-oxo-3,4-dihydro-1,5-benzothiazepin-5(2H)-essigsäure-tert.-butylester.

A) 20,5 g Dimethylphosphonoessigsäure-tert.-butylester wurden mit 25 g 4-Fluorphenoxyethylbromid nach der in Beispiel 5 A) beschriebenen Methode umgesetzt. Das Reaktionsgemisch wurde wie in Beispiel 5 A) beschrieben aufgearbeitet. Es wurden 20,4 g 4-(4-Fluorphenoxy)-2-(dimethylphosphono)-n-buttersäure-tert.-butylester erhalten.
B) 20,4 g des vorstehend erhaltenen Produktes wurden mit 4,8 g Paraformaldehyd nach der im Beispiel 5 B) beschriebenen Methode umgesetzt. Das Reaktionsgemisch wurde wie in Beispiel 5 B) beschrieben aufgearbeitet. Als Rohprodukt wurden 15,3 g öliges α-[2-(4-Fluorphenoxy)-ethyl]-acrylsäure-tert.-butylester erhalten. Dieses wurde ohne weitere chromatographische Reinigung in der nächsten Stufe weiterverarbeitet.
C) 15,3 g des vorstehend erhaltenen Produktes wurden mit 5,1 ml Cyclopentancarbonsäure nach der in Beispiel 5 C) beschriebenen Methode umgesetzt. Das Reaktionsgemisch wurde wie in Beispiel 5 C) beschrieben aufgearbeitet. Es wurden 6,0 g 1-[2'-(tert.-Butoxycarbonyl)-4'-(4-fluorphenoxy)-butyl]-cyclopentan-1-carbonsäure mit einem Schmelzpunkt von 58 - 63 °C und weitere 7,6 g öliges, noch leicht verunreinigtes Produkt erhalten.
D) Zu einer Lösung von 100 ml 1-Fluor-2-nitrobenzol in 1800 ml Ethanol wurde eine Lösung von 122,4 g N-Acetyl-L-cystein und 181,9 g Natriumhydrogencarbonat in 550 ml Wasser gegeben. Das Reaktionsgemisch wurde 3 Stunden unter Rückfluß gekocht, dann auf Raumtemperatur abgekühlt und von dem Niederschlag abfiltriert. Das Filtrat wurde auf ca. 700 ml eingeengt und der verbleibende Rückstand wurde in 1,8 l Wasser aufgenommen. Die wäßrige Phase wurde mit Diethylether extrahiert und anschließend durch Zugabe von konzentrierter, wäßriger Salzsäure-Lösung auf pH 1 eingestellt. Es fiel ein gelber Feststoff aus, welcher abgesaugt wurde. Es wurden 253,6 g rohes R-(2-Nitrophenyl)-N-acetyl-L-cystein erhalten, welches ohne weitere Reinigung weiterverarbeitet wurde.
E) 253,6 g des vorstehend erhaltenen Produktes wurden mit 825 ml 18-molarer Schwefelsäure und 3,3 l Wasser versetzt. Das Reaktionsgemisch wurde 40 Minuten am Rückfluß erhitzt und dann auf 0 °C abgekühlt. Es wurden 1925 ml konzentrierte wäßrige Ammoniak-Lösung zugefügt. Der hierbei ausgefallene Feststoff wurde abgesaugt und aus Wasser umkristallisiert. Es wurden 143 g R-(2-Nitrophenyl)-L-cystein erhalten.
F) 100 g des vorstehend erhaltenen Produktes und 62,2 g Kaliumcarbonat wurden in 7 l Wasser gelöst. Anschließend wurden innerhalb von 3 Stunden 120 g Carbethoxyphthalimid portionsweise zugefügt und das Reaktionsgemisch wurde weitere 5 Stunden gerührt und noch mehrere Stunden stehengelassen. Anschließend wurde der ausgefallene Feststoff abgesaugt und das Filtrat wurde mit konzentrierter wäßriger Salzsäure-Lösung auf einen pH-Wert von 2 bis 3 gebracht. Der hierbei ausgefallene Niederschlag wurde abgesaugt, mehrmals mit Wasser gewaschen und anschließend mit ca. 1 l Ethanol unter leichter Erwärmung (ca. 40 °C) aufgeschlämmt. Nach Abkühlen wurde der Feststoff abgesaugt und an der Luft getrocknet. Es wurden 100 g (2R)-3-(2-Nitrophenylthio)-2-phthalimidopropionsäure erhalten, welche ohne weitere Reinigung weiterverarbeitet wurden.
G) 100 g des vorstehend erhaltenen Produktes wurden in 1,5 l Methanol aufgeschlemmt. Dazu wurden 0,8 g Palladium/Kohle (5 %ig)-Katalysator gegeben und das Reaktionsgemisch wurde 5 Stunden hydriert. Anschließend wurde der Katalysator abgetrennt und das Lösungsmittel unter vermindertem Druck eingedampft. Es wurden 71,6 g rohes (2R)-3-(2-Aminophenylthio)-2-phthalimidopropionsäure als gelbbraunes Öl erhalten, welches ohne weitere Reinigung weiterverarbeitet wurde.
H) 71,6 g des vorstehend erhaltenen Produktes wurden in Dimethylformamid gelöst. Zu der Lösung wurden 38,0 g 1-[3-(Dimethylamino)-propyl]-3-ethylcarbodiimid-hydrochlorid gegeben und das Reaktionsgemisch wurde 3 Stunden bei Raumtemperatur gerührt. Anschließend wurde das Reaktionsgemisch mit 1,5 l Essigsäureethylester verdünnt und mehrmals mit je 1,5 l 1-normaler wäßriger Natriumhydrogencarbonat-Lösung extrahiert. Die organische Phase wurde anschließend zweimal mit je 200 ml Wasser gewaschen, über Magnesiumsulfat getrocknet und unter vermindertem Druck zur Trockene eingeengt. Der Rückstand wurde mittels Flash-Säulenchromatographie unter Verwendung von Essigsäureethylester/Cyclohexan (1:1) als Elutionsmittel gereinigt. Es wurden 46,3 g (3R)-2,3-Dihydro-3-phthalimido-1,5-benzothiazepin-4(5H)-on erhalten.
I) Zu einer Lösung von 46,3 g des vorstehend erhaltenen Produktes in 300 ml Tetrahydrofuran wurden 10,6 g pulverisiertes Kaliumhydroxid und 4,8 g Tetrabutylammoniumbromid gegeben. Das Reaktionsgemisch wurde auf 0 °C abgekühlt und dann wurden langsam 23,2 ml Bromessigsäure-tert.-butylester hinzugetropft. Das Reaktionsgemisch wurde noch weitere 3 Stunden bei Raumtemperatur gerührt. Dann wurde filtriert und das Filtrat unter vermindertem Druck zur Trockene eingeengt. Der Rückstand wurde in Diethylether aufgenommen und die Etherphase wurde mit Wasser und 1-molarer Kaliumhydrogensulfat-Lösung gewaschen, über Magnesiumsulfat getrocknet und anschließend unter vermindertem Druck eingeengt. Das verbleibende ölige Rohprodukt wurde mit Essigsäureethylester und Diethylether versetzt. Der gebildete Niederschlag wurde abgesaugt. Es wurden 34 g (3R)-5-(tert.-Butoxycarbonylmethyl)-2,3-dihydro-3-phthalimido-1,5-benzothiazepin-4(5H)-on als Feststoff erhalten. Nach Einengen der Mutterlauge unter vermindertem Druck wurde nochmals 25 g leicht verunreinigtes öliges Produkt erhalten.
   Optischer Drehwert [α]²⁰_{D} = -146 °C (c = 0,8 in Dichlormethan)
J) 2 g des vorstehend erhaltenen Produktes wurden mit 7,5 ml Ethanolamin versetzt und das Gemisch wurde 10 Minuten bei 80 °C gerührt. Anschließend wurde die Heizquelle entfernt und noch weitere 30 Minuten gerührt. Sodann wurde das Reaktionsgemisch zur Aufarbeitung mit 70 ml 5 %-iger wäßriger Natriumchlorid-Lösung versetzt und das erhaltene Gemisch mit Toluol extrahiert. Die organische Phase wurde abgetrennt, über Natriumsulfat getrocknet und unter vermindertem Druck zur Trockene eingeengt. Es wurden 1,46 g rohes noch toluolhaltiges (3R)-3-Amino-4-oxo-3,4-dihydro-1,5-benzothiazepin-5(2H)-essigsäure-tert-butylester als toluolhaltiger Feststoff erhalten.
K) Zu 100 ml trockenem Dichlormethan wurden 1,45 g des vorstehenden Produktes, 1,75 g des unter C) erhaltenen Cyclopentancarbonsäurederivates, 0,70 g Hydroxybenzotriazol und 1,50 ml N-Methylmorpholin gegeben. Dann wurde das Reaktionsgemisch auf 0 °C abgekühlt und es wurden 1,76 g 1-Ethyl-3-(3-dimethylaminopropyl)-carbodiimid-hydrochlorid zugefügt und das Reaktionsgemisch wurde weitere 5 Stunden bei Raumtemperatur gerührt. Zur Aufarbeitung wurde das Reaktionsgemisch mit 1-molarer Kaliumhydrogensulfat-Lösung versetzt, die organische Phase wurde abgetrennt und mit 1-molarer Kaliumhydrogencarbonat-Lösung und gesättigter Natriumchlorid-Lösung gewaschen, über Natriumsulfat getrocknet und unter vermindertem Druck zur Trockene eingeengt. Das erhaltene Rohprodukt wurde durch Flash-Säulenchromatographie unter Verwendung von n-Hexan/Essigsäureethylester (4:1) als Elutionsmittel gereinigt. Es wurden 1,9 g der Titelverbindung als Öl erhalten.
   - IR-Spektrum (als Film):: 3366 cm⁻¹, 3059 cm⁻¹, 2969 cm⁻¹, 2874 cm⁻¹, 1727 cm⁻¹, 1657 cm⁻¹, 1505 cm⁻¹.

### Beispiel 13:

### (3R)-3-{1-[2'-Carboxy-4'-(4-fluorphenoxy)-butyl]-cyclopentan-1-carbonylamino}-4-oxo-3,4-dihydro-1,5-benzothiazepin-5 (2H)-essigsäure.

1,9 g (3R)-3-{1-[2'-(tert.-Butoxycarbonyl)-4'-(4-fluorphenoxy)-butyl]-cyclopentan-1-carbonylamino}-4-oxo-3,4-dihydro-1,5-benzothiazepin-5(2H)-essigsäure-tert.-butylester ( Herstellung siehe Beispiel 12) wurden nach der in Beispiel 6 beschriebenen Methode mit Trifluoressigsäure hydrolysiert. Das Reaktionsgemisch wurde wie in Beispiel 6 beschrieben aufgearbeitet. Es wurden 0,56 g der Titelverbindung als amorpher Feststoff erhalten, Schmelzpunkt 90 - 94 °C.

### Beispiel 14:

### (3R)-3-{1-[2'-(tert.-Butoxycarbonyl)-5'-(3,4-dimethoxyphenyl)-pentyl]-cyclopentan-1-carbonylamino}-4-oxo-3,4-dihydro-1,5-benzothiazepin-5(2H)-essigsäure-tert.-butylester

A) 6,7 g Triphenylphosphin wurden in 200 ml Acetonitril gelöst. Nach Abkühlen der Lösung auf 0 °C wurden 1,3 ml Brom zugetropft. Dann wurde das Kühlbad entfernt und es wurde eine Lösung von 5 g 3-(3,4-Dimethoxyphenyl)-1-propanol in 80 ml Acetonitril zugetropft. Das Reaktionsgemisch wurde anschließend unter Rückfluß erhitzt, wobei mit Hilfe eines Wasserabscheiders mehrmals innerhalb von 6 Stunden jeweils 10 ml Destillat entnommen und die entnommene Menge durch frisches Acetonitril ergänzt wurden. Zur Aufarbeitung wurde das Lösungsmittel unter vermindertem Druck abgedampft und der verbleibende Rückstand wurde in Diethylether aufgenommen und filtriert. Das Filtrat wurde unter vermindertem Druck eingeengt und durch Flash-Säulenchromatographie unter Verwendung von Cyclohexan/Methyl-tert.-butylether (7:2) gereinigt. Es wurden 5,5 g 3-(3,4-Dimethoxyphenyl-1-brom-propan als farbloses Öl erhalten.
B) 5,5 g des vorstehend erhaltenen Produktes wurden nach der in Beispiel 5 A) beschriebenen Methode mit 3,8 ml Dimethylphosphonoessigsäure-tert.-butylester umgesetzt. Das Reaktionsgemisch wurde wie in Beispiel 5 A) beschrieben aufgearbeitet. Es wurden 6,1 g 4-(3,4-Dimethoxyphenyl)-2-(dimethylphosphono)-valeriansäure-tert.-butylester als farbloses Öl erhalten.
C) 6 g des vorstehend erhaltenen Produktes wurden nach der in Beispiel 5 B) beschriebenen Methode mit Paraformaldehyd umgesetzt. Das Reaktionsgemisch wurde wie in Beispiel 5 B) beschrieben aufgearbeitet. Das erhaltene Rohprodukt wurde durch Flash-Säulenchromatographie unter Verwendung von Methyl-tert.-butylether/Cyclohexan (1:3) als Elutionsmittel gereinigt. Es wurden 3,4 g öliger 1-[3-(3,4-Dimethoxyphenyl)-propyl]-acrylsäure-tert.-butylester erhalten.
D) 3,4 g des vorstehend erhaltenen Produktes wurden mit 1,3 ml Cyclopentancarbonsäure nach der in Beispiel 5 C) beschriebenen Methode umgesetzt. Das Reaktionsgemisch wurde wie in Beispiel 5 C) beschrieben aufgearbeitet. Das Rohprodukt wurde durch Flash-Säulenchromatographie unter Verwendung von Essigsäureethylester/Cyclohexan (1:3) als Elutionsmittel gereinigt. Es wurden 2,5 g ölige 1-[2-(tert.-Butoxycarbonyl)-5-(3,4-dimethoxyphenyl)-pentyl]-cyclopentancarbonsäure erhalten.
E) 2,5 g des vorstehend erhaltenen Produktes wurden in 50 ml Acetonitril gelöst. Bei einer Temperatur von 0 °C wurden unter Feuchtigkeitsausschluß zu der Lösung nacheinander 4,2 ml Diisopropylethylamin, 1,7 g 2-Chlor-1-methylpyridiniumjodid und 2,5 g (3R)-3-Amino-4-oxo-3,4-dihydro-1,5-benzodiazepin-5(2H)-essigsäure-tert.-butylester (Herstellung siehe Beispiel 12 J) zugegeben. Das Reaktionsgemisch wurde noch 30 Minuten bei 0 °C und 2 Stunden bei Raumtemperatur gerührt. Zur Aufarbeitung wurde das Reaktionsgemisch unter vermindertem Druck zur Trockene eingeengt und der verbleibende Rückstand wurde in Dichlormethan gelöst. Die Lösung wurde zunächst mit verdünnter wäßriger Salzsäurelösung und dann mit Wasser geschüttelt. Die organische Phase wurde abgetrennt und die wäßrige Phase wurde noch 2 mal mit Dichlormethan extrahiert. Anschließend wurden die vereinigten organischen Phasen über Natriumsulfat getrocknet und unter vermindertem Druck eingeengt. Als Rückstand wurden 3 g der Titelverbindung als Öl erhalten.
   Dünnschichtchromatographie an Kieselgel: Rf-Wert = 0,4 (Eluens Cyclohexan/Essigsäurethylester 1:1)

### Beispiel 15:

### (3R)-3-{1-(2'-Carboxy-5'-(3,4-dimethoxyphenyl)-pentyl]-cyclopentan-1-carbonylamino}-4-oxo-3,4-dihydro-1,5-benzothiazepin-5(2H)-essigsäure

3 g (3R)-3-{1-[2'-tert.-Butoxycarbonyl)-5'-(3,4-dimethoxyphenyl)-pentyl]-cyclopentan-1-carbonylamino}-4-oxo-3,4-dihydro-1,5-benzothiazepin-5(2H)-essigsäure-tert.-butylester (Herstellung siehe Beispiel 14) wurden in 20 ml Dichlormethan gelöst. Zu der Lösung wurden 3 ml Trifluoressigsäure gegeben und das Reaktionsgemisch wurde 2 Tage bei Raumtemperatur gerührt. Zur Aufarbeitung wurde das Reaktionsgemisch unter vermindertem Druck eingeengt. Zur völligen Entfernung von Trifluoressigsäure wurde der Rückstand mehrmals mit jeweils 2 ml Toluol versetzt und wieder eingedampft. Das so erhaltenen Rohprodukt wurde durch Flash-Chromatographie an Kieselgel gereinigt, wobei als Elutionsmittel zunächst Dichlormethan/Essigsäureethylester 1:1 und dann reiner Essigsäureethylester verwendet wurde. Nach Einengen des Eluates unter vermindertem Druck wurden 1,26 g der Titelverbindung als amorpher Feststoff erhalten.
- IR-Spektrum als KBr-Preßling:: 3365 cm⁻¹, 2942 cm⁻¹, 1726 cm⁻¹, 1652 cm⁻¹.

### Beispiel 16:

### 3-{1-[2'-(tert.-Butoxycarbonyl)-4'-phenyl-butyl]-cyclopentan-1-carbonylamino}-2,3,4,5-tetrahydro-2-oxo-1H-1-benzazepin-1-essigsäure-benzylester.

A) 10,5 g 3-Amino-2,3,4,5-tetrahydro-2-oxo-1H-1-benzazepin-1-essigsäure-tert.-butylester (Herstellung siehe Beispiel 1 I), 8,25 g p-Toluolsulfonsäure-Hydrat und 20,1 ml Benzylalkohol wurden in 174 ml Toluol gegeben. Das Reaktionsgemisch wurde 4 Stunden am Wasserabscheider gekocht, wobei ein ursprünglich ausgefallener Niederschlag langsam in Lösung ging. Danach wurde das Toluol unter vermindertem Druck abgezogen und der verbleibende Rückstand wurde mit Methyl-tert.-butylether gerührt und dann abfiltriert. Der so erhaltene feste Rückstand wurde in Dichlormethan gelöst und die Lösung wurde durch Zugabe von wäßriger Natriumcarbonat-Lösung unter Eiskühlung alkalisch gestellt. Danach wurde die Dichlormethanphase abgetrennt, mit Wasser gewaschen, über Natriumsulfat getrocknet und eingedampft. Das erhaltene Rohprodukt wurde zur Reinigung aus Methyl-tert.-butylether umkristallisiert. Es wurden 8,2 g 3-Amino-2,3,4,5-tetrahydro-2-oxo-1H-1-benzazepin-1-essigsäurebenzylester mit einem Schmelzpunkt von 105 bis 107 °C erhalten.
B) 12,8 g des vorstehend erhaltenen Produktes wurden mit 13,7 g 1-[2'-(tert.-Butoxycarbonyl)-4'-phenyl-butyl]-cyclopentan-1-carbonsäure (Herstellung siehe Beispiel 5C)) nach der in Beispiel 3C) beschriebenen Methode umgesetzt. Das Reaktionsgemisch wurde wie in Beispiel 3C) beschrieben, aufgearbeitet. Es wurden 19,3 g der Titelverbindung mit einem Schmelzpunkt von 118 bis 123 °C erhalten.

### Beispiel 17:

### 3-[1-(2'-Carboxy-4'-phenyl-butyl)-cyclopentan-1-carbonylamino]-2,3,4,5-tetrahydro-2-oxo-lH-l-benzazepin-l-essigsäure-benzylester.

15 g 3-{1-[2'-tert.-Butoxycarbonyl)-4'-phenyl-butyl]-cyclopentan-1-carbonylamino}-2,3,4,5-tetrahydro-2-oxo-1H-1-benzazepin-1-essigsäurebenzylester (Herstellung siehe Beispiel 16) wurden nach der in Beispiel 6 beschriebenen Methode mit 56 ml Trifluoressigsäure umgesetzt. Das Reaktionsgemisch wurde wie in Beispiel 6 beschrieben aufgearbeitet und das erhaltene Rohprodukt wurde aus Methyl-tert.-butylether kristallisiert. Es wurden 13,1 g der Titelverbindung mit einem Schmelzpunkt von 86 bis 90 °C erhalten.

### Beispiel 18:

### 3-{1-[2'-(tert.-Butylcarbonyloxymethoxycarbonyl)-4'-phenylbutyl]-cyclopentan-1-carbonylamino}-2,3,4,5-tetrahydro-2-oxo-1H-1-benzazepin-1-essigsäure-benzylester.

2 g 3-[1-(2'-Carboxy-4'-phenyl-butyl)-cyclopentan-1-carbonylamino] -2,3,4,5-tetrahydro-2-oxo-1H-1-benzazepin-1-essigsäurebenzylester (Herstellung siehe Beispiel 17) wurden unter Feuchtigkeitsausschluß in 20 ml trockenem Dichlormethan gelöst. Zu der Lösung wurden 0,46 ml Triethylamin und 0,1 g Dimethylaminopyridin gegeben. Dann wurde unter Eiskühlung eine Lösung von 0,5 g Pivalinsäurechlormethylester in 3 ml trockenem Dichlormethan zugetropft. Das Reaktionsgemisch wurde anschließend 2 Tage bei Raumtemperatur gerührt. Zur Aufarbeitung wurde das Reaktionsgemisch auf Wasser gegeben, die organische Phase abgetrennt, mit wäßriger Natriumbicarbonat-Lösung und anschließend mit Wasser gewaschen, über Natriumsulfat getrocknet und unter vermindertem Druck eingeengt. Das als Rückstand verbleibende Rohprodukt wurde durch Flash-Chromatographie an 150 g Kieselgel gereinigt, wobei als Elutionsmittel ein n-Hexan/Essigsäureethylester-Gemisch mit einer Zusammensetzung von zunächst 7:3 und dann 1:1 eingesetzt wurde. Es wurden 1,1 g reines 3-{1-[2'-(Pivaloyloxymethoxycarbonyl)-4'-phenyl-butyl]-cyclopentan-1-carbonylamino}-2,3,4,5-tetrahydro-2-oxo-1H-1-benzazepin-1-essigsäurebenzylester als fester Schaum mit einem Schmelzbereich von 71 - 78 °C erhalten.

### Beispiel 19:

### 3-{1-[2'-(Pivaloyloxymethoxycarbonyl)-4'-phenyl-butyl]-cyclopentan-1-carbonylamino}-2,3,4,5-tetrahydro-2-oxo-1H-1-benzazepin-1-essigsäure.

1,0 g 3-{1-[2'-(Pivaloyloxymethoxycarbonyl)-4'-phenyl-butyl]cyclopentan-1-carbonylamino}-2,3,4,5-tetrahydro-2-oxo-1H-1-benzazepin-1-essigsäure-benzylester (Herstellung siehe Beispiel 18) wurden in 100 ml Ethanol gelöst. Die Lösung wurde mit 0,5 g Palladium/Kohle-Katalysator (5 %-ig) versetzt. Dann wurde 3 Stunden mit einem Wasserstoffdruck von 5 bar hydriert. Zur Aufarbeitung wurde vom Katalysator abfiltriert und die filtrierte Lösung eingedampft. Der erhaltene Rückstand wurde bei 80 °C unter vermindertem Druck getrocknet. Es wurden 0,7 g der Titelverbindung als glasartiges Produkt erhalten.
- IR-Spektrum (als KBr-Preßling) :: 3410 cm⁻¹, 1750 cm⁻¹, 1660 cm⁻¹

Nach den in den vorstehenden Beispielen beschriebenen Verfahren können auch die in der nachfolgenden Tabelle 1 aufgeführten Verbindungen der Formel I hergestellt werden.

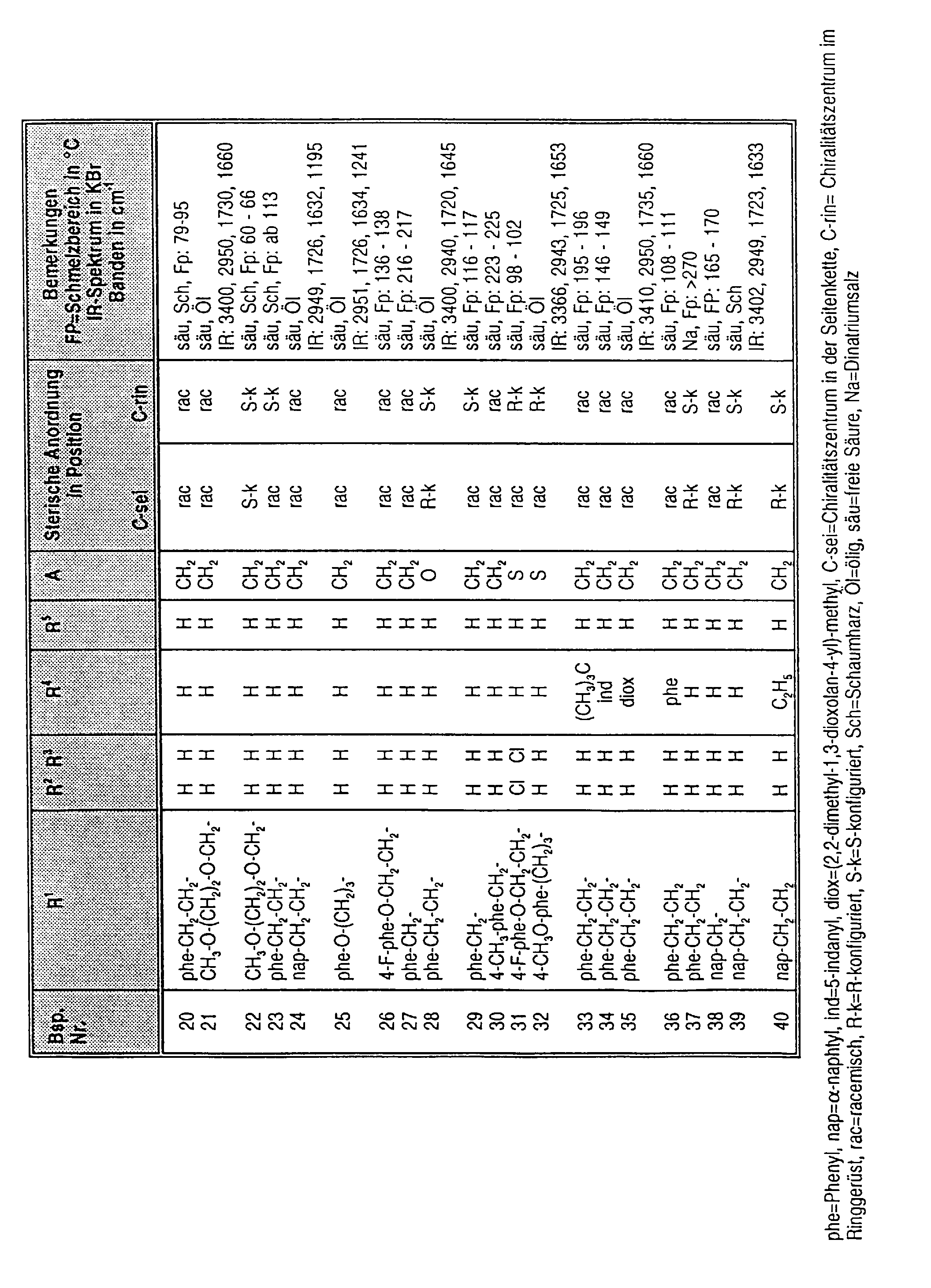

### Beispiel I:

### (3S,2'R)-3-{1-[2'-(Ethoxycarbonyl)-4'-phenyl-butyl]-cyclopentan-1-carbonylamino}-2,3,4,5-tetrahydro-2-oxo-1H-1-benzazepin-1-essigsäure enthaltende Tabletten

Man stellte Tabletten in folgender Zusammensetzung pro Tablette her:

| | |
|---|---|
| (3S,2'R)-3-{1-[2'-(Ethoxycarbonyl)-4'-phenyl-butyl]-cyclopentan-1-carbonylamino}-2,3,4,5-tetrahydro-2-oxo-1H-1-benzazepin-1-essigsäure | 20 mg |
| Maisstärke | 60 mg |
| Milchzucker | 135 mg |
| Gelatine (als 10 %-ige Lösung) | 6 mg |

Der Wirkstoff, die Maisstärke und der Milchzucker wurden mit der 10 %-igen Gelatine-Lösung eingedickt. Die Paste wurde zerkleinert, und das entstehende Granulat wurde auf ein geeignetes Blech gebracht und bei 45 °C getrocknet. Das getrocknete Granulat wurde durch eine Zerkleinerungsmaschine geleitet und in einem Mixer mit weiteren folgenden Hilfsstoffen vermischt:

| | |
|---|---|
| Talkum | 5 mg |
| Magnesiumstearat | 5 mg |
| Maisstärke | 9 mg |

und sodann zu Tabletten von 240 mg verpreßt.

### Beispiel II:

### (3S,2'R)-3-[1-(2'-Carboxy-4'-phenyl-butyl)-cyclopentan-1-carbonylamino]-2,3,4,5-tetrahydro-2-oxo-1H-1-benzazepin-1-essigsäure enthaltende Injektionslösung.

Man stellte eine Injektionslösung mit folgender Zusammensetzung pro 5 ml her:

| | |
|---|---|
| (3S,2'R)-3-[1-(2'-Carboxy-4'-phenyl-butyl)-cyclopentan-1-carbonylamino]-2,3,4,5-tetrahydro-2-oxo-1H-1-benzazepin-1-essigsäure | 10 mg |
| Na₂HPO₄•7H₂O | 43,24 mg |
| NaH₂PO₄•2H₂O | 7,72 mg |
| NaCl | 30,0 mg |
| gereinigtes Wasser | 4948,0 mg |

Die Feststoffe wurden in Wasser gelöst, die Lösung wurde sterilisiert und in Portionen von jeweils 5 ml in Ampullen abgefüllt.

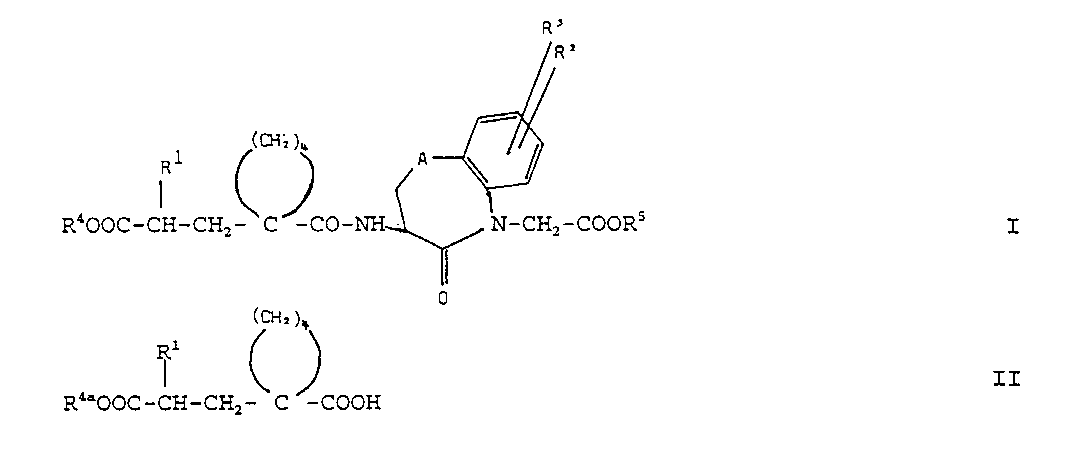

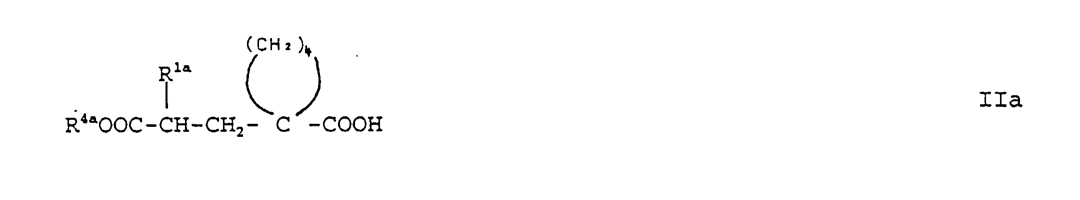

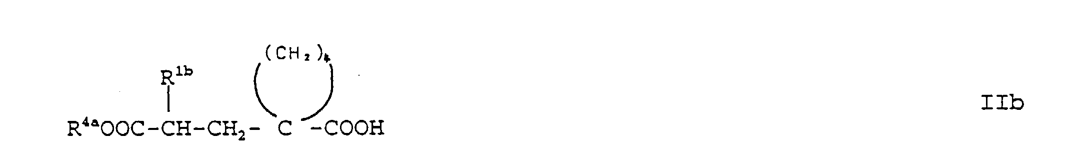

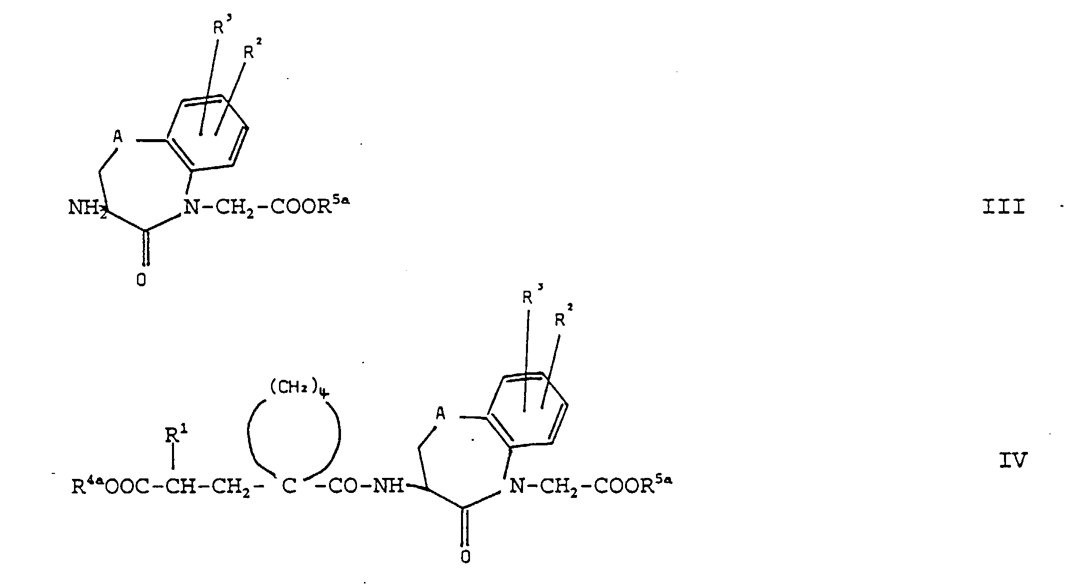

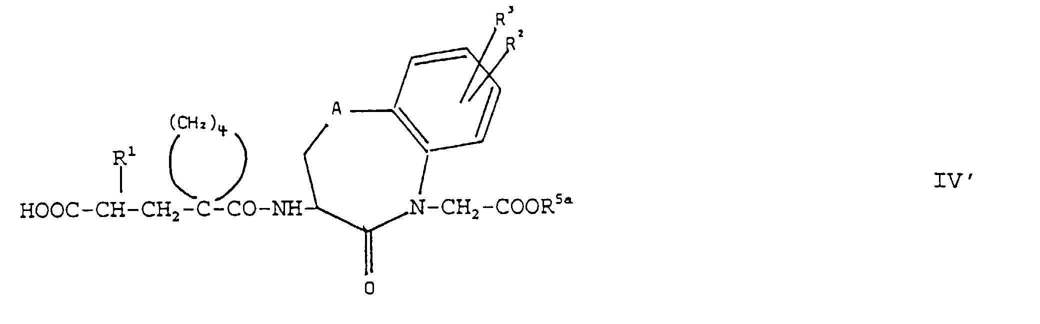

R⁶-OH V

R⁶-X Va

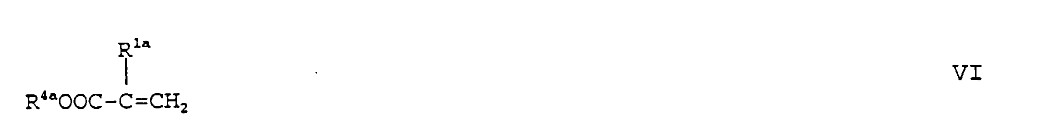

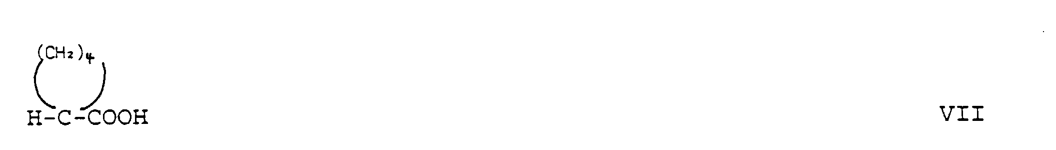

R^{4a}OOC-CH₂-CH₂-Y VIII

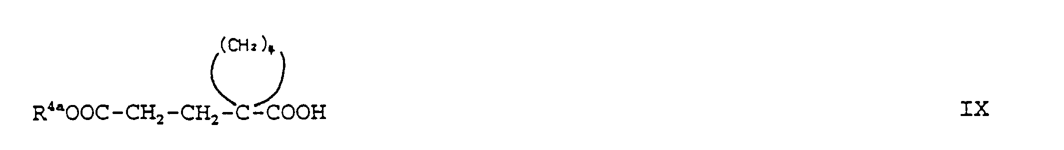

R^{1a}-X Xa

R^{1b}-X Xb

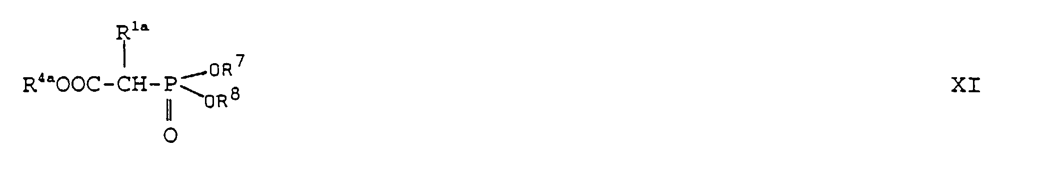

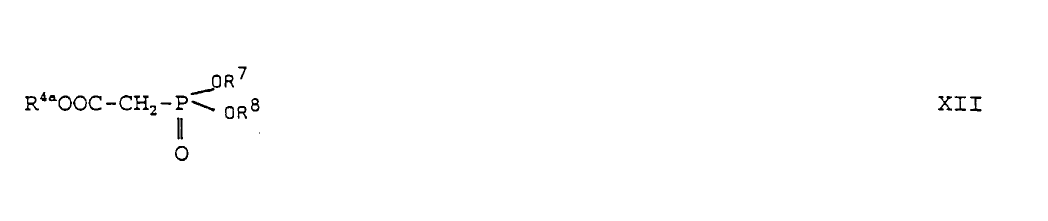

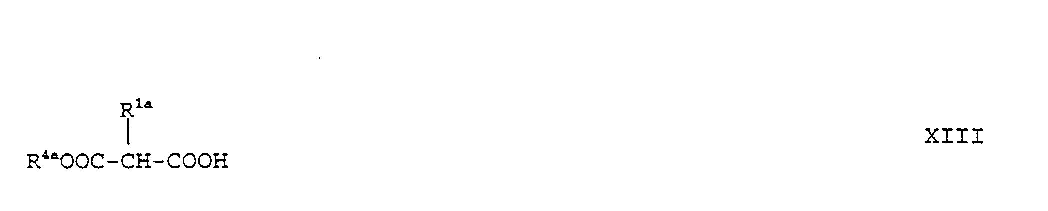

R^{4a}OOC-CH₂-COOR⁹ XIV

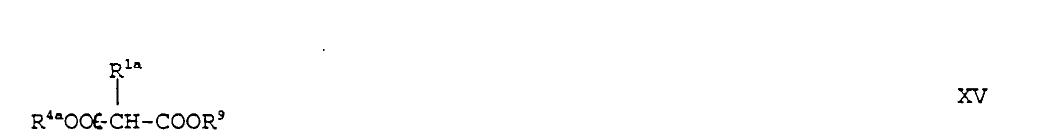

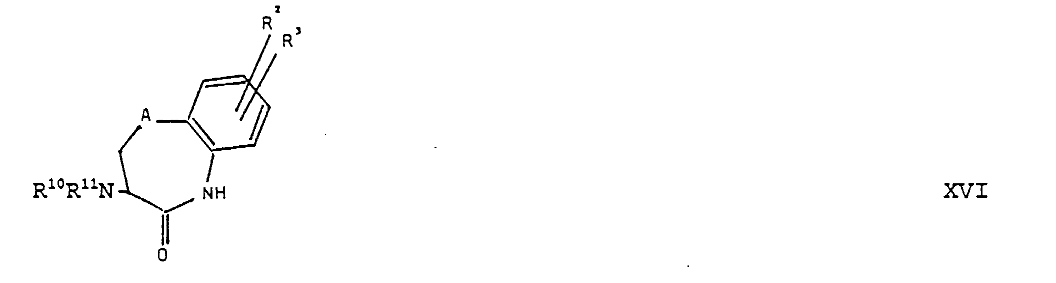

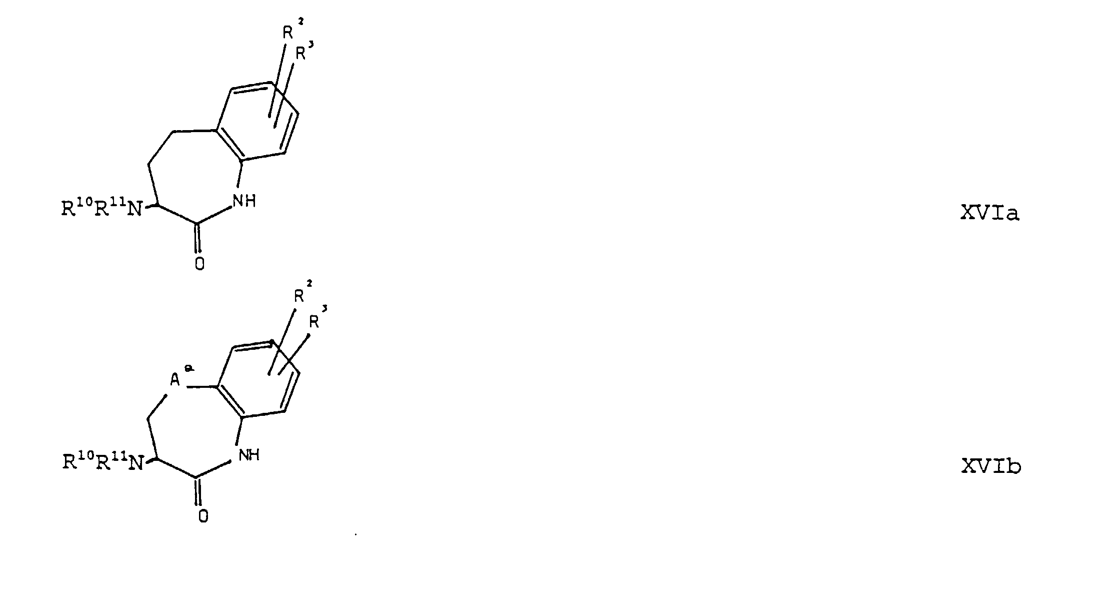

X-CH₂-COOR^{5a} XVII

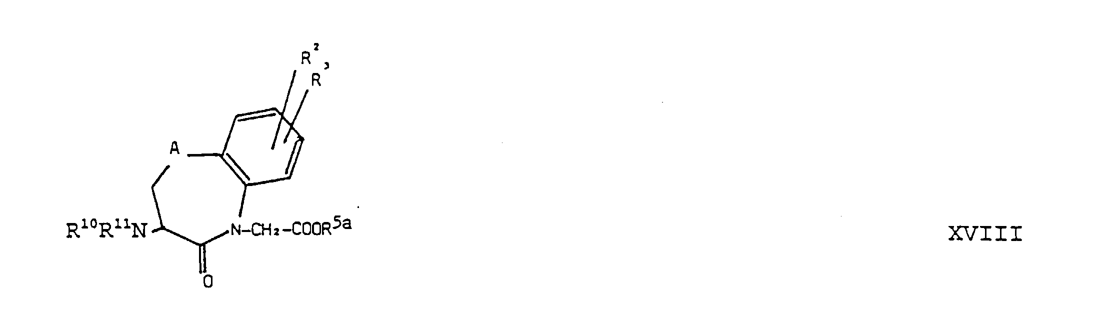

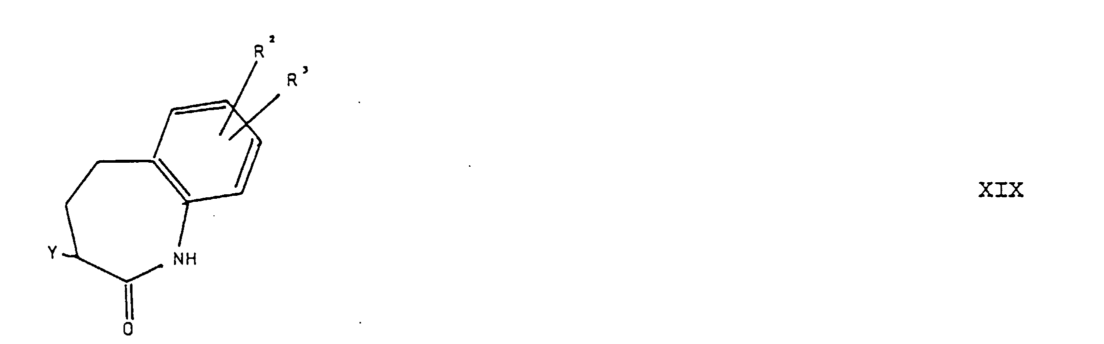

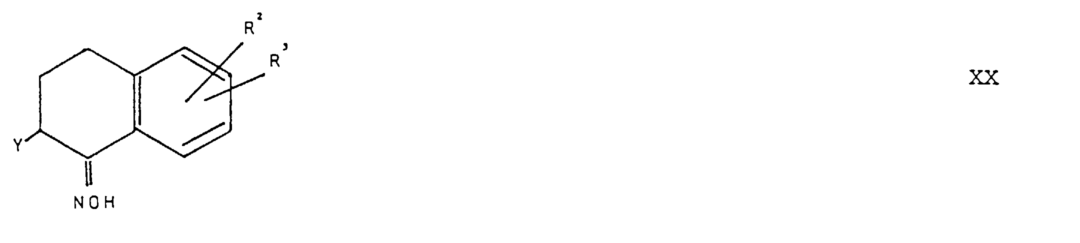

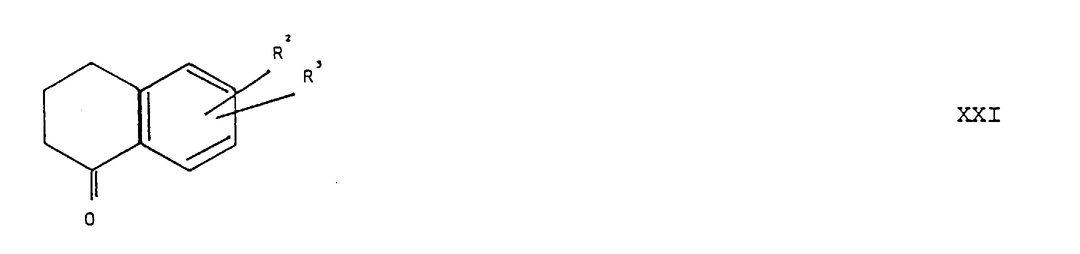

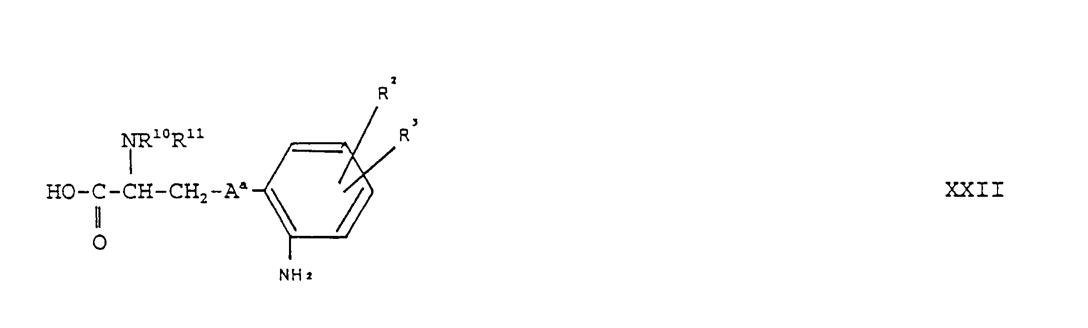

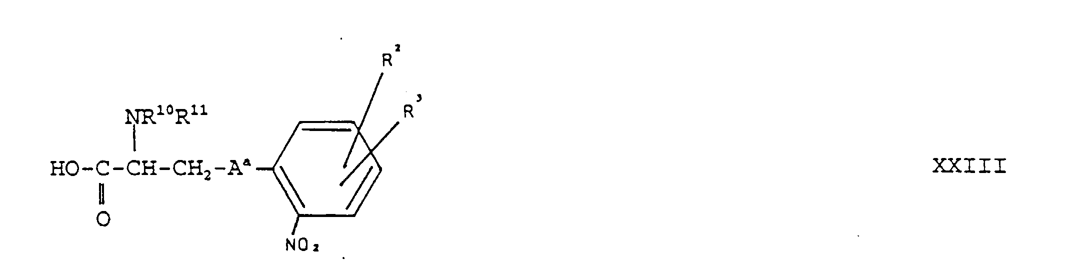

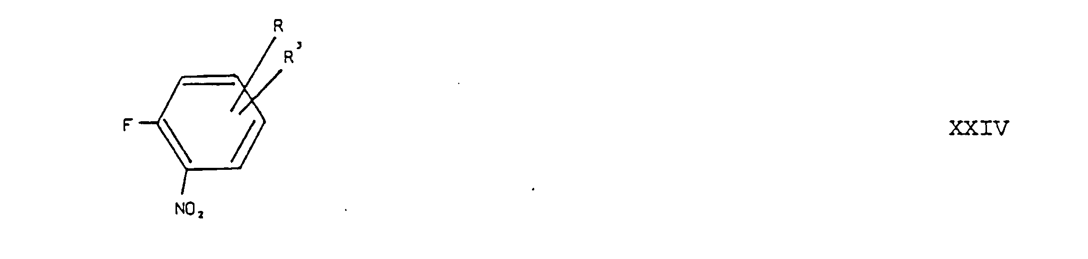

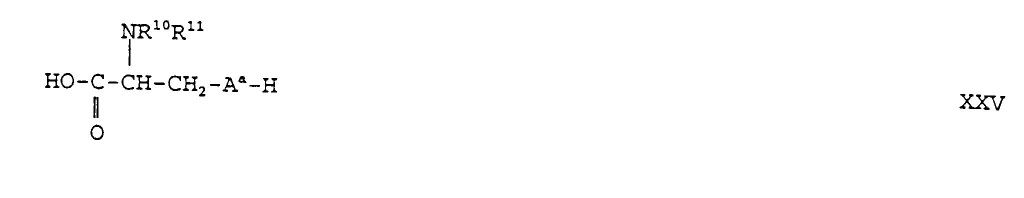

## Patentansprüche

1. Verbindungen der allgemeinen Formel I 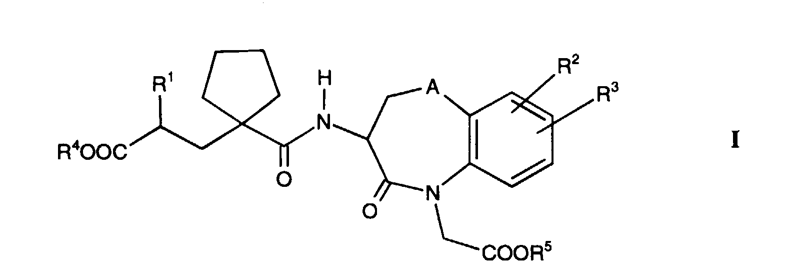 worin
R¹ für eine C₁₋₄-Alkoxy-C₁₋₄-alkylgruppe, deren C₁₋₄-Alkoxyrest durch eine C₁₋₄-Alkoxygruppe substituiert ist, eine Phenyl-C₁₋₄-alkyl- oder Phenyloxy-C₁₋₄-alkylgruppe, welche gegebenenfalls im Phenylring durch C₁₋₄-Alkyl, C₁₋₄-Alkoxy oder Halogen substituiert sein kann, oder eine Naphthyl-C₁₋₄-alkylgruppe steht,
A für CH₂, O oder S steht,
R² Wasserstoff oder Halogen bedeutet,
R³ Wasserstoff oder Halogen bedeutet,
R⁴ Wasserstoff oder eine einen biolabilen Ester bildende Gruppe bedeutet und
R⁵ Wasserstoff oder eine einen biolabilen Ester bildende Gruppe bedeutet
und physiologisch verträgliche Salze der Säuren der Formel I.

2. Verbindungen gemäß Anspruch 1, worin R⁴ und/oder R⁵ eine einen biolabilen Ester bildende Gruppe bedeuten.

3. Verbindungen nach einem der vorstehenden Ansprüche, worin die einen biolabilen Ester bildende Gruppe eine C₁₋₄-Alkylgruppe darstellt, eine gegebenenfalls im Phenylring durch C₁₋₄-Alkyl oder durch eine an 2 benachbarte Kohlenstoffatome gebundene C₃₋₄-Alkylenkette substituierte Phenyl- oder Phenyl-C₁₋₃-alkylgruppe, insbesondere Phenyl, Benzyl oder Indanyl, darstellt, eine im Dioxolanring gegebenfalls durch C₁₋₄-Alkyl substituierte Dioxolanylmethylgruppe, insbesondere (2,2-Dimethyl-1,3-dioxolan-4-yl)methyl, darstellt, oder eine gegebenenfalls an der Oxymethylgruppe durch C₁₋₄-Alkyl substituierte C₂-C₆-Alkanoyloxymethylgruppe darstellt.

4. Verbindungen nach einem der vorstehenden Ansprüche dadurch gekennzeichnet, daß R⁴ eine einen biolabilen Ester bildende Gruppe bedeutet und R⁵ Wasserstoff ist.

5. Verbindungen nach einem der vorstehenden Ansprüche, dadurch gekennzeichnet, daß A für CH₂ steht.

6. Verbindungen gemäß Anspruch 5, worin R¹ eine Phenethylgruppe oder eine Naphthylethylgruppe und R² Wasserstoff bedeuten.

7. Arzneimittel, enthaltend eine pharmakologisch wirksame Menge einer Verbindung gemäß Anspruch 1 und übliche pharmazeutische Hilfs- und/oder Trägerstoffe.

8. Verfahren zur Herstellung von Verbindungen der allgemeinen Formel I 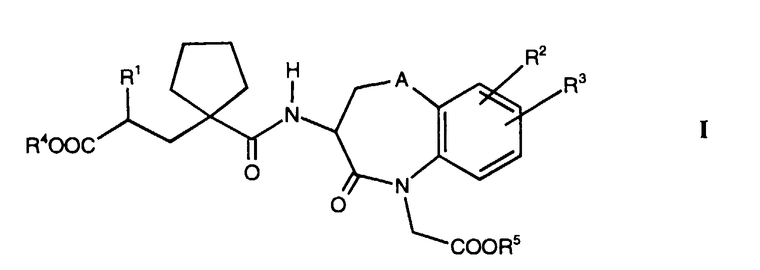 worin
R¹ für eine C₁₋₄-Alkoxy-C₁₋₄-alkylgruppe, deren C₁₋₄-Alkoxyrest durch eine C₁₋₄-Alkoxygruppe substituiert ist, eine Phenyl-C₁₋₄-alkyl- oder Phenyloxy-C₁₋₄-alkylgruppe, welche gegebenenfalls im Phenylring durch C₁₋₄-Alkyl, C₁₋₄-Alkoxy oder Halogen substituiert sein kann, oder eine Naphthyl-C₁₋₄-alkylgruppe steht,
A für CH₂, O oder S steht,
R² Wasserstoff oder Halogen bedeutet,
R³ Wasserstoff oder Halogen bedeutet,
R⁴ Wasserstoff oder eine einen biolabilen Ester bildende Gruppe bedeutet und
R⁵ Wasserstoff oder eine einen biolabilen Ester bildende Gruppe bedeutet
und physiologisch verträglichen Salzen der Säuren der Formel I,
dadurch gekennzeichnet, daß man
Säuren der allgemeinen Formel II 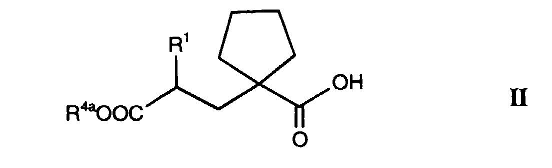 worin R¹ obige Bedeutung besitzt und R^{4a} eine Säureschutzgruppe bedeutet, oder deren reaktionsfähige Säurederivate mit Aminen der allgemeinen Formel III 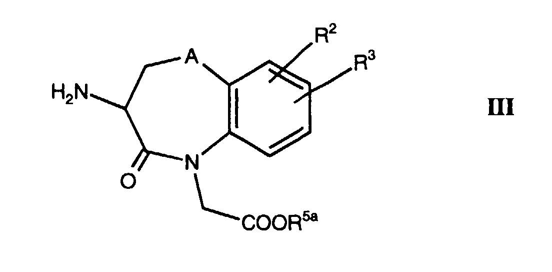 worin R², R³ und A obige Bedeutung besitzen und R^{5a} eine Säureschutzgruppe bedeutet, zu Amiden der allgemeinen Formel IV, 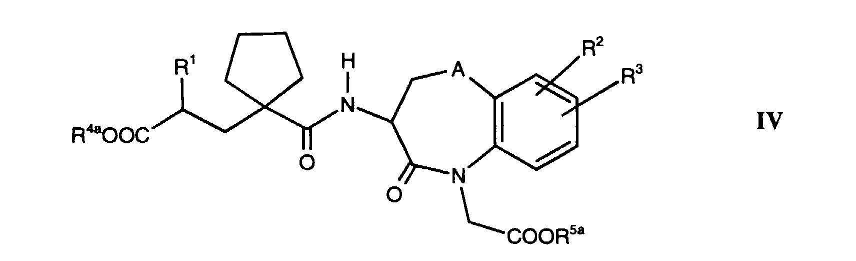 worin R¹, R², R³, R^{4a}, R^{5a}, und A obige Bedeutung besitzen, umsetzt, und in den Verbindungen der Formel IV die Säureschutzgruppen R^{4a} und R^{5a}, sofern diese nicht eine gewünschte einen biolabilen Ester bildende Gruppe darstellen, gleichzeitig oder in beliebiger Reihenfolge nacheinander abspaltet
und gewünschtenfalls jeweils die freiwerdende Säuregruppe mit einem Alkohol der allgemeinen Formel V oder einem entsprechenden reaktiven Derivat der allgemeinen Formel Va
R⁶-OH (V)
R⁶-X (Va)
worin R⁶ eine einen biolabilen Ester bildende Gruppe darstellt und X eine abspaltbare reaktive Gruppe bedeutet, verestert,
und gewünschtenfalls erhaltene Säuren der Formel I in ihre physiologisch verträglichen Salze überführt oder Salze der Säuren der Formel I in die freien Säuren überführt.

## Claims

1. Compounds of the general formula I: 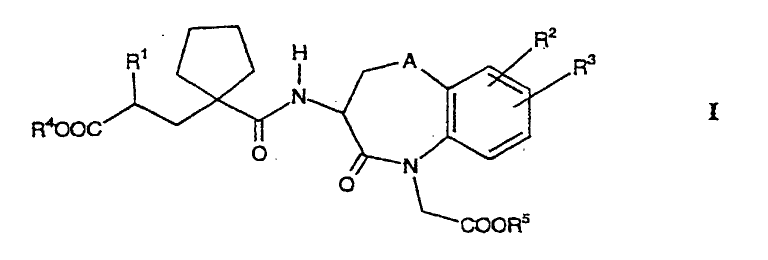 in which
R¹ is a C₁-C₄ alkoxy-C₁-C₄ alkyl group whose C₁-C₄ alkoxy radical is substituted by a C₁-C₄ alkoxy group, or a phenyl-C₁-C₄ alkyl or phenyloxy-C₁-C₄ alkyl group which can optionally be substituted in the phenyl ring by C₁-C₄ alkyl, C₁-C₄ alkoxy or halogen, or a naphthyl-C₁-C₄ -alkyl group,
A is CH₂, O or S,
R² is hydrogen or halogen,
R³ is hydrogen or halogen,
R⁴ is hydrogen or a group forming a biolabile ester, and
R⁵ is hydrogen or a group forming a biolabile ester,
and physiologically tolerated salts of the acids of formula I.

2. Compounds according to Claim 1, in which R⁴ and/or R⁵ are a group forming a biolabile ester.

3. Compounds according to any preceding claim, in which the group forming a biolabile ester is a C₁-C₄ alkyl group, a phenyl or phenyl- C₁-C₃ alkyl group which is optionally substituted in the phenyl ring by C₁-C₄ alkyl or by a C₃-C₄ alkylene chain bonded to two adjacent carbon atoms, in particular phenyl, benzyl or indanyl, a dioxolanylmethyl group which is optionally substituted in the dioxolane ring by C₁-C₄ alkyl, in particular (2,2-dimethyl-1,3-dioxolan-4-yl)methyl, or a C₂-C₆-alkanoyloxymethyl group which is optionally substituted on the oxymethyl group by C₁-C₄ alkyl.

4. Compounds according to any preceding claim, characterised in that R⁴ is a group forming a biolabile ester, and R⁵ is hydrogen.

5. Compounds according to any preceding claim, characterised in that A is CH₂.

6. Compounds according to Claim 5, in which R¹ is a phenethyl group or a naphthylethyl group and R² is hydrogen.

7. A medicament containing a pharmacologically effective amount of a compound according to Claim 1 and conventional pharmaceutical auxiliary substances and/or vehicles.

8. A process for the preparation of compounds of the general formula I: 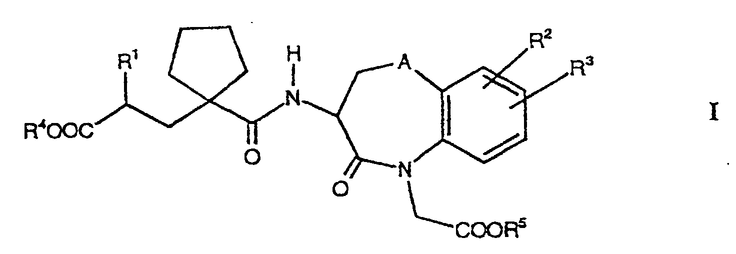 in which
R¹ is a C₁-C₄ alkoxy-C₁-C₄ alkyl group whose C₁-C₄ alkoxy radical is substituted by a C₁-C₄ alkoxy group, or a phenyl-C₁-C₄ alkyl or phenyloxy-C₁-C₄ alkyl group which can optionally be substituted in the phenyl ring by C₁-C₄ alkyl, C₁-C₄ alkoxy or halogen, or a naphthyl-C₁-C₄ alkyl group,
A is CH₂, O or S,
R² is hydrogen or halogen,
R³ is hydrogen or halogen,
R⁴ is hydrogen or a group forming a biolabile ester, and
R⁵ is hydrogen or a group forming a biolabile ester,
and physiologically tolerated salts of the acids of the formula I, characterised in that acids of the general formula II: 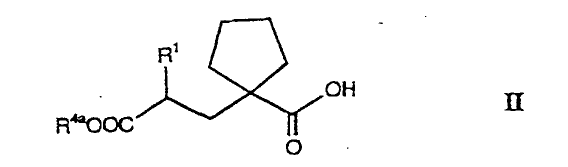 in which R¹ has the above meaning and R^{4a} is an acid protective group, or the reactive acid derivatives thereof is reacted with amines of the general formula III: 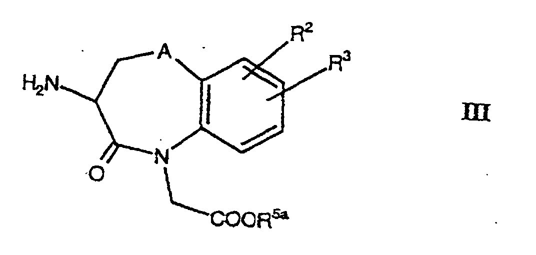 in which R², R³ and A have the above meaning, and R^{5a} is an acid protective group, to give amides of the general formula IV: 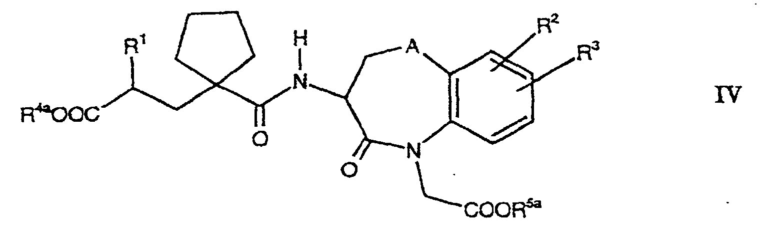 in which R¹, R², R³, R^{4a}, R^{5a} and A have the above meaning, and the acid protective groups R^{4a} and R^{5a}, unless they are a desired group forming a biolabile ester, in the compounds of the formula IV are eliminated simultaneously or successively in any desired sequence,
and, if desired, in each case the unblocked acid group is esterified with an alcohol of the general formula V or a corresponding reactive derivative of the general formula Va:
R⁶-OH (V)
R⁶-X (Va)
in which R⁶ is a group forming a biolabile ester and X is a reactive group which can be eliminated,
and, if desired, resulting acids of formula I are converted into their physiologically tolerated salts, or salts of the acids of formula I are converted into the free acids.

## Revendications

1. Composés de formule générale I 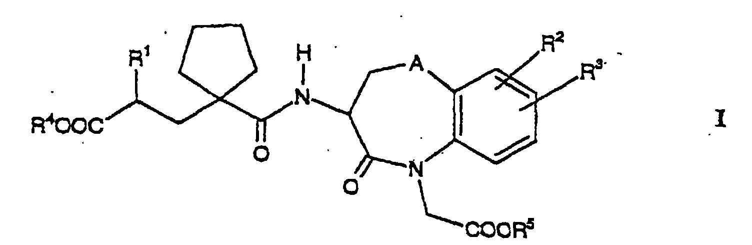 dans laquelle
R¹ représente un groupe alcoxy en C₁ à C₄-alkyle en C₁ à C₄, dont le radical alcoxy en C₁ à C₄ est substitué par un groupe alcoxy en C₁ à C₄, un groupe phényl-alkyle en C₁ à C₄ ou phényloxy-alkyle en C₁ à C₄, qui peut éventuellement être substitué dans le noyau phényle par un alkyle en C₁ à C₄ , un alcoxy en C₁ à C₄ ou un halogène, ou un groupe naphtyle en C₁ à C₄,
A représente CH₂, O ou S,
R² représente un hydrogène ou un halogène,
R³ représente un hydrogène ou un halogène,
R⁴ représente un hydrogène ou un groupe formant un ester biolabile et
R⁵ représente un hydrogène ou un groupe formant un ester biolabile,
et les sels physiologiquement acceptables des acides de formule I.

2. Composés selon la revendication 1, dans lesquels R⁴ et/ou R⁵ représentent également un groupe formant un ester biolabile.

3. Composés selon l'une des revendications précédentes, dans lesquels le groupe formant un ester biolabile représente un groupe alkyle en C₁ à C₄, un groupe phényle ou phényl-alkyle en C₁ à C₃ éventuellement substitué dans le noyau phényle par un alkyle en C₁ à C₄ ou par une chaîne alkylène en C₃ à C₄ liée sur deux atomes de carbone voisins, en particulier un phényle, un benzyle ou un indanyle, un groupe dioxolanylméthyle éventuellement substitué dans le noyau dioxolane par un alkyle en C₁ à C₄, en particulier un (2,2-diméthyl-1,3-dioxolan-4-yl)méthyle, ou un groupe alcanoyloxy en C₂ à C₆-méthyle éventuellement substitué sur le groupe oxyméthyle par un alkyle en C₁ à C₄.

4. Composés selon l'une des revendications précédentes, caractérisés en ce que R⁴ représente un groupe formant un ester biolabile et R₅ est un hydrogène.

5. Composés selon l'une des revendications précédentes, caractérisés en ce que A représente CH₂.

6. Composés selon la revendication 5, dans lesquels R¹ représente un groupe phénéthyle ou un groupe naphtyléthyle et R² représente un hydrogène.

7. Médicament contenant une quantité pharmacologiquement active d'un composé selon la revendication 1 et des adjuvants et/ou supports pharmaceutiques habituels.

8. Procédé de préparation de composés de formule générale I 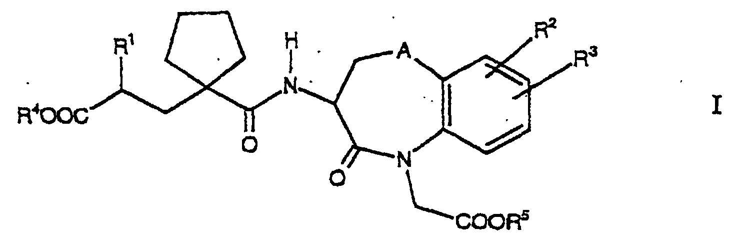 dans laquelle
R¹ représente un groupe alcoxy en C₁ à C₄-alkyle en C₁ à C₄ dont le radical alcoxy en C₁ à C₄ est substitué par un groupe alcoxy en C₁ à C₄, un groupe phényl-alkyle en C₁ à C₄ ou phényloxy-alkyle en C₁ à C₄, qui peut le cas échéant être substitué dans le noyau phényle par un alkyle en C₁ à C₄, un alcoxy en C₁ à C₄ ou un halogène, ou un groupe naphtyl-alkyle en C₁ à C₄,
A représente CH₂, O ou S,
R² représente un hydrogène ou un halogène,
R³ représente un hydrogène ou un halogène,
R⁴ représente un hydrogène ou un groupe formant un ester biolabile et
R⁵ représente un hydrogène ou un groupe formant un ester biolabile
et de sels physiologiquement acceptables des acides de formule I,
caractérisé en ce que
l'on fait réagir des acides de formule générale II 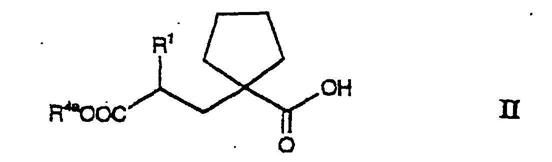 dans laquelle R¹ a la signification donnée ci-dessus et R^{4a} représente un groupe protecteur d'acide, ou leurs dérivés acides réactifs, avec des amines de formule générale III, 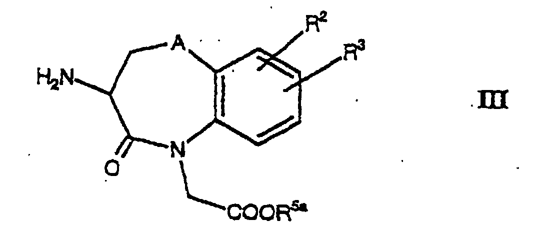 dans laquelle R², R³ et A ont la signification donnée ci-dessus et R^{5a} représente un groupe protecteur d'acide, pour donner des amides de formule générale IV, 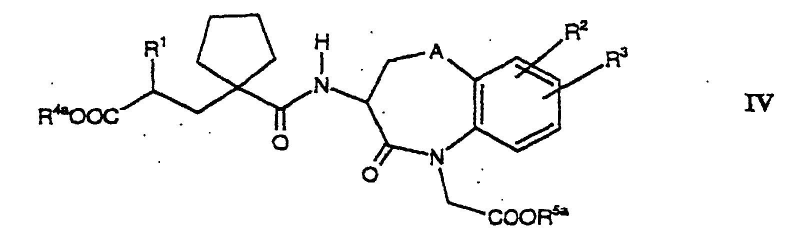 dans laquelle R¹, R², R³, R^{4a}, R^{5a} et A ont la signification donnée ci-dessus, et en ce que dans les composés de formule IV on sépare simultanément ou successivement dans un ordre quelconque les groupes protecteurs d'acide R^{4a} et R^{5a}, dans la mesure où ils ne représentent pas un groupe formant un ester biolabile désiré,
et si on le désire on estérifie respectivement le groupe acide qui se libère avec un alcool de formule générale V ou un dérivé réactif correspondant de formule générale Va
R⁶-OH (V)
R⁶-X (Va)
dans laquelle R⁶ représente un groupe formant un ester biolabile et X représente un groupe réactif séparable,
et si on le désire on transforme les acides de formule I obtenus en leurs sels physiologiquement acceptables, ou on transforme les sels des acides de formule I en les acides libres.
